# FASCICULE DE BREVET EUROPEEN

(11) **EP 1 877 558 B1**
(45) Date de publication et mention de la délivrance du brevet: **03.08.2016**
(21) Numéro de dépôt: 06743758.2
(22) Date de dépôt: 27.04.2006
(51) Int. Cl.: C12N 15/45, C07K 14/115, C07K 14/135, C07K 14/12, A61K 39/155

(54) **PREPARATION DE COMPLEXES SOLUBLES PROTEINE N-PROTEINE P TRONQUEE OU DE PROTEINE N SOLUBLE D'UN VIRUS DE LA FAMILLE DES PARAMYXOVIRIDAE ET LEURS UTILISATIONS VACCINALES**
HERSTELLUNG VON LÖSLICHEN KOMPLEXEN BESTEHEND AUS N-PROTEIN UND VERKÜRZTEM P-PROTEIN ODER LÖSLICHEM N-PROTEIN AUS DER FAMILIE DER PARAMYXOVIRIDAE UND DEREN VERWENDUNGEN ALS IMPFSTOFFE
PREPARATION OF SOLUBLE N-PROTEIN/TRUNCATED P-PROTEIN COMPLEXES OR N-PROTEINS SOLUBLE IN A VIRUS OF THE PARAMYXOVIRIDAE FAMILY AND USE THEREOF IN VACCINES

(30) Priorité: 29.04.2005 FR 0504426
(43) Date de publication de la demande: 16.01.2008
(73) Titulaire: INSTITUT NATIONAL DE LA RECHERCHE AGRONOMIQUE (INRA), 75007 Paris Cedex 07 (FR)
(72) Inventeur: ELEOUËT, Jean-François, F-91650 Breuillet (FR); RIFFAULT, Sabine, F-78350 Jouy-en-Josas (FR)
(74) Mandataire: Colombet, Alain André
(86) Numéro de dépôt international: PCT/FR2006/000949
(87) Numéro de publication internationale: WO 2006/117456

(56) Documents cités:
- WO-A-2004/087062
- CASTAGNE NATHALIE ET AL: "Biochemical characterization of the respiratory syncytial virus P-P and P-N protein complexes and localization of the P protein ollgomerization domain" JOURNAL OF GENERAL VIROLOGY, vol. 85, no. Part 6, juin 2004 (2004-06), pages 1643-1653, XP002361372 ISSN: 0022-1317 cité dans la demande
- SLACK M S ET AL: "Characterization of the interaction of the human respiratory syncytial virus phosphoprotein and nucleocapsid protein using the two-hybrid system" VIRUS RESEARCH, AMSTERDAM, NL, vol. 55, no. 2, juin 1998 (1998-06), pages 167-176, XP002234289 ISSN: 0168-1702 cité dans la demande
- KHATTAR SUNIL K ET AL: "Mapping the domains on the phosphoprotein of bovine respiratory syncytial virus required for N-P and P-L interactions using a minigenome system" JOURNAL OF GENERAL VIROLOGY, vol. 82, no. 4, avril 2001 (2001-04), pages 775-779, XP002361379 ISSN: 0022-1317 cité dans la demande
- CHOUDHARY SURESH K ET AL: "Characterization of the oligomerization domain of the phosphoprotein of human parainfluenza virus type 3" VIROLOGY, vol. 302, no. 2, 25 octobre 2002 (2002-10-25), pages 373-382, XP002361373 ISSN: 0042-6822
- BANKAMP B ET AL: "Domains of the measles virus N protein required for binding to P protein and self-assembly" VIROLOGY 1996 UNITED STATES, vol. 216, no. 1, 1996, pages 272-277, XP002361375 ISSN: 0042-6822
- CHAN Y P ET AL: "Mapping of domains responsible for nucleocapsid protein-phosphoprotein interaction of Henipaviruses." THE JOURNAL OF GENERAL VIROLOGY. JUN 2004, vol. 85, no. Pt 6, juin 2004 (2004-06), pages 1675-1684, XP002361376 ISSN: 0022-1317
- MITRA-KAUSHIK SHIBANI ET AL: "Identification of a cytotoxic T-cell epitope on the recombinant nucleocapsid proteins of Rinderpest and Peste des petits ruminants viruses presented as assembled nucleocapsids", VIROLOGY, vol. 279, no. 1, 5 January 2001 (2001-01-05), pages 210-220, ISSN: 0042-6822
- TAN WEN SIANG ET AL: "Solubility, immunogenicity and physical properties of the nucleocapsid protein of Nipah virus produced in Escherichia coli", JOURNAL OF MEDICAL VIROLOGY, vol. 73, no. 1, May 2004 (2004-05), pages 105-112, ISSN: 0146-6615
- RIFFAULT SABINE ET AL: 'A new subunit vaccine based on nucleoprotein nanoparticles confers partial clinical and virological protection in calves against bovine respiratory syncytial virus.' VACCINE vol. 28, no. 21, 07 Mai 2010, pages 3722 - 3734 ISSN: 1873-2518
- Shibani Mitra-Kaushik ET AL: "Identification of a Cytotoxic T-Cell Epitope on the Recombinant Nucleocapsid Proteins of Rinderpest and Peste des petits ruminants Viruses Presented as Assembled Nucleocapsids", Virology, vol. 279, no. 1, 1 January 2001 (2001-01-01), pages 210-220, XP055112835, ISSN: 0042-6822, DOI: 10.1006/viro.2000.0698
- Wen Siang Tan ET AL: "Solubility, immunogenicity and physical properties of the nucleocapsid protein of Nipah virus produced inEscherichia coli", Journal of Medical Virology, vol. 73, no. 1, 1 May 2004 (2004-05-01), pages 105-112, XP055057712, ISSN: 0146-6615, DOI: 10.1002/jmv.20052

### SEQUENCE LISTING

<110> INRA
<120> Préparation de complexes solubles protéine N - fragment de la protéine P ou de protéine N soluble d'un virus de la famille des Paramyxoviridae et leurs utilisations vaccinales
<130> BET 06P0332
<150> FR 0504426
   <151> 2005-04-29
<160> 26
<170> PatentIn version 3.3
<210> 1
   <211> 241
   <212> PRT
   <213> Human respiratory syncytial virus
<220>
   <221> MISC_FEATURE
   <222> (1)..(241)
   <223> phosphoprotein P (Swissprot P12579)
<400> 1
   Met Glu Lys Phe Ala Pro Glu Phe His Gly Glu Asp Ala Asn Asn Arg
   1               5                   10                  15
   Ala Thr Lys Phe Leu Glu Ser Ile Lys Gly Lys Phe Thr Ser Pro Lys
               20                  25                  30
   Asp Pro Lys Lys Lys Asp Ser Ile Ile Ser Val Asn Ser Thr Asp Ile
           35                  40                  45
   Glu Val Thr Lys Glu Ser Pro Ile Thr Ser Asn Ser Thr Ile Ile Asn
       50                  55                  60
   Pro Thr Asn Glu Thr Asp Asp Asn Ala Gly Asn Lys Pro Asn Tyr Gln
   65                  70                  75                  80
   Arg Lys Pro Leu Val Ser Phe Lys Glu Asp Pro Ile Pro Ser Asp Asn
                   85                  90                  95
   Pro Phe Ser Lys Leu Tyr Lys Glu Thr Ile Glu Thr Phe Asp Asn Asn
               100                 105                 110
   Glu Glu Glu Ser Ser Tyr Ser Tyr Glu Glu Ile Asn Asp Gln Thr Asn
           115                 120                 125
   Asp Asn Ile Thr Ala Arg Leu Asp Arg Ile Asp Glu Lys Leu Ser Glu
   130 135 140
   Ile Leu Gly Met Leu His Thr Leu Val Val Ala Ser Ala Gly Pro Thr
   145 150 155 160
   Ser Ala Arg Asp Gly Ile Arg Asp Ala Met Val Gly Leu Arg Glu Glu
   165 170 175
   Met Ile Glu Lys Ile Arg Thr Glu Ala Leu Met Thr Asn Asp Arg Leu
   180 185 190
   Glu Ala Met Ala Arg Leu Arg Asn Glu Glu Ser Glu Lys Met Ala Lys
   195 200 205
   Asp Thr Ser Asp Glu Val Ser Leu Asn Pro Thr Ser Glu Lys Leu Asn
   210 215 220
   Asn Leu Leu Glu Gly Asn Asp Ser Asp Asn Asp Leu Ser Leu Glu Asp
   225 230 235 240
   Phe
<210> 2
   <211> 568
   <212> PRT
   <213> Sendai virus
<220>
   <221> MISC_FEATURE
   <222> (1)..(568)
   <223> phosphoprotein P (Swissprot P04859)
<400> 2
   Met Asp Gln Asp Ala Phe Ile Leu Lys Glu Asp Ser Glu Val Glu Arg
   1 5 10 15
   Glu Ala Pro Gly Gly Arg Glu Ser Leu Ser Asp Val Ile Gly Phe Leu
   20 25 30
   Asp Ala Val Leu Ser Ser Glu Pro Thr Asp Ile Gly Gly Asp Arg Ser
   35 40 45
   Trp Leu His Asn Thr Ile Asn Thr Pro Gln Gly Pro Gly Ser Ala His
   50 55 60
   Arg Ala Lys Ser Glu Gly Glu Gly Glu Val Ser Thr Pro Ser Thr Gln
   65                  70                  75                  80
   Asp Asn Arg Ser Gly Glu Glu Ser Arg Val Ser Gly Arg Thr Ser Lys
                   85                  90                  95
   Pro Glu Ala Glu Ala His Ala Gly Asn Leu Asp Lys Gln Asn Ile His
               100                 105                 110
   Arg Ala Phe Gly Gly Arg Thr Gly Thr Asn Ser Val Ser Gln Asp Leu
           115                 120                 125
   Gly Asp Gly Gly Asp Ser Gly Ile Leu Glu Asn Pro Pro Asn Glu Arg
       130                 135                 140
   Gly Tyr Pro Arg Ser Gly Ile Glu Asp Glu Asn Arg Glu Met Ala Ala
   145                 150                 155                 160
   His Pro Asp Lys Arg Gly Glu Asp Gln Ala Glu Gly Leu Pro Glu Glu
                   165                 170                 175
   Val Arg Gly Gly Thr Ser Leu Pro Asp Glu Gly Glu Gly Gly Ala Ser
               180                 185                 190
   Asn Asn Gly Arg Ser Met Glu Pro Gly Ser Ser His Ser Ala Arg Val
           195                 200                 205
   Thr Gly Val Leu Val Ile Pro Ser Pro Glu Leu Glu Glu Ala Val Leu
       210                 215                 220
   Arg Arg Asn Lys Arg Arg Pro Thr Asn Ser Gly Ser Lys Pro Leu Thr
   225                 230                 235                 240
   Pro Ala Thr Val Pro Gly Thr Arg Ser Pro Pro Leu Asn Arg Tyr Asn
                   245                 250                 255
   Ser Thr Gly Ser Pro Pro Gly Lys Pro Pro Ser Thr Gln Asp Glu His
               260                 265                 270
   Ile Asn Ser Gly Asp Thr Pro Ala Val Arg Val Lys Asp Arg Lys Pro
           275                 280                 285
   Pro Ile Gly Thr Arg Ser Val Ser Asp Cys Pro Ala Asn Gly Arg Pro
       290                 295                 300
   Ile His Pro Gly Leu Glu Ser Asp Ser Thr Lys Lys Gly Ile Gly Glu
   305                 310                 315                 320
   Asn Thr Ser Ser Met Lys Glu Met Ala Thr Leu Leu Thr Ser Leu Gly
                   325                 330                 335
   Val Ile Gln Ser Ala Gln Glu Phe Glu Ser Ser Arg Asp Ala Ser Tyr
               340                 345                 350
   Val Phe Ala Arg Arg Ala Leu Lys Ser Ala Asn Tyr Ala Glu Met Thr
           355                 360                 365
   Phe Asn Val Cys Gly Leu Ile Leu Ser Ala Glu Lys Ser Ser Ala Arg
       370                 375                 380
   Lys Val Asp Glu Asn Lys Gln Leu Leu Lys Gln Ile Gln Glu Ser Val
   385                 390                 395                 400
   Glu Ser Phe Arg Asp Ile Tyr Lys Arg Phe Ser Glu Tyr Gln Lys Glu
                   405                 410                 415
   Gln Asn Ser Leu Leu Met Ser Asn Leu Ser Thr Leu His Ile Ile Thr
               420                 425                 430
   Asp Arg Gly Gly Lys Thr Asp Asn Thr Asp Ser Leu Thr Arg Ser Pro
           435                 440                 445
   Ser Val Phe Ala Lys Ser Lys Glu Asn Lys Thr Lys Ala Thr Arg Phe
       450                 455                 460
   Asp Pro Ser Met Glu Thr Leu Glu Asp Met Lys Tyr Lys Pro Asp Leu
   465                 470                 475                 480
   Ile Arg Glu Asp Glu Phe Arg Asp Glu Ile Arg Asn Pro Val Tyr Gln
                   485                 490                 495
   Glu Arg Asp Thr Glu Pro Arg Ala Ser Asn Ala Ser Arg Leu Leu Pro
               500                 505                 510
   Ser Lys Glu Lys Pro Thr Met His Ser Leu Arg Leu Val Ile Glu Ser
           515                 520                 525
   Ser Pro Leu Ser Arg Ala Glu Lys Ala Ala Tyr Val Lys Ser Leu Ser
       530                 535                 540
   Lys Cys Lys Thr Asp Gln Glu Val Lys Ala Val Met Glu Leu Val Glu
   545 550 555 560
   Glu Asp Ile Glu Ser Leu Thr Asn
   565
<210> 3
   <211> 507
   <212> PRT
   <213> Measles virus
<220>
   <221> MISC_FEATURE
   <222> (1)..(507)
   <223> Phsophosprotein P (Swissprot CAA91364)
<400> 3
   Met Ala Glu Glu Gln Ala Arg His Val Lys Asn Gly Leu Glu Cys Ile
   1 5 10 15
   Arg Ala Leu Lys Ala Glu Pro Ile Gly Ser Leu Ala Val Glu Glu Ala
   20 25 30
   Met Ala Ala Trp Ser Glu Ile Ser Asp Asn Pro Gly Gln Asp Arg Ala
   35 40 45
   Thr Cys Lys Glu Glu Glu Ala Gly Ser Ser Gly Leu Ser Lys Pro Cys
   50 55 60
   Leu Ser Ala Ile Gly Ser Thr Glu Gly Gly Ala Pro Arg Ile Arg Gly
   65 70 75 80
   Gln Gly Ser Gly Glu Ser Asp Asp Asp Ala Glu Thr Leu Gly Ile Pro
   85 90 95
   Ser Arg Asn Leu Gln Ala Ser Ser Thr Gly Leu Gln Cys Tyr His Val
   100 105 110
   Tyr Asp His Ser Gly Glu Ala Val Lys Gly Ile Gln Asp Ala Asp Ser
   115 120 125
   Ile Met Val Gln Ser Gly Leu Asp Gly Asp Ser Thr Leu Ser Gly Gly
   130 135 140
   Asp Asp Glu Ser Glu Asn Ser Asp Val Asp Ile Gly Glu Pro Asp Thr
   145 150 155 160
   Glu Gly Tyr Ala Ile Thr Asp Arg Gly Ser Ala Pro Ile Ser Met Gly
   165 170 175
   Phe Arg Ala Ser Asp Val Glu Thr Ala Glu Gly Gly Glu Ile His Glu
   180 185 190
   Leu Leu Lys Leu Gln Ser Arg Gly Asn Asn Phe Pro Lys Leu Gly Lys
   195 200 205
   Thr Leu Asn Val Pro Pro Pro Pro Asn Pro Ser Arg Ala Ser Thr Ser
   210 215 220
   Glu Thr Pro Ile Lys Lys Gly Thr Asp Ala Arg Leu Ala Ser Phe Gly
   225 230 235 240
   Thr Glu Ile Ala Ser Leu Leu Thr Gly Gly Ala Thr Gln Cys Ala Arg
   245 250 255
   Lys Ser Pro Ser Glu Pro Ser Gly Pro Gly Ala Pro Ala Gly Asn Val
   260 265 270
   Pro Glu Cys Val Ser Asn Ala Ala Leu Ile Gln Glu Trp Thr Pro Glu
   275 280 285
   Ser Gly Thr Thr Ile Ser Pro Arg Ser Gln Asn Asn Glu Glu Gly Gly
   290 295 300
   Asp Tyr Tyr Asp Asp Glu Leu Phe Ser Asp Val Gln Asp Ile Lys Thr
   305 310 315 320
   Ala Leu Ala Lys Ile His Glu Asp Asn Gln Lys Ile Ile Ser Lys Leu
   325 330 335
   Glu Ser Leu Leu Leu Leu Lys Gly Glu Val Glu Ser Ile Lys Lys Gln
   340 345 350
   Ile Asn Arg Gln Asn Ile Ser Ile Ser Thr Leu Glu Gly His Leu Ser
   355 360 365
   Ser Ile Met Ile Ala Ile Pro Gly Leu Gly Lys Asp Pro Asn Asp Pro
   370 375 380
   Thr Ala Asp Val Glu Leu Asn Pro Asp Leu Lys Pro Ile Ile Gly Arg
   385 390 395 400
   Asp Ser Gly Arg Ala Leu Ala Glu Val Leu Lys Lys Pro Val Ala Ser
   405 410 415
   Arg Gln Leu Gln Gly Met Thr Asn Gly Arg Thr Ser Ser Arg Gly Gln
   420 425 430
   Leu Leu Lys Glu Phe Gln Leu Lys Pro Ile Gly Lys Lys Val Ser Ser
   435 440 445
   Ala Val Gly Phe Val Pro Asp Thr Gly Pro Ala Ser Arg Ser Val Ile
   450 455 460
   Arg Ser Ile Ile Lys Ser Ser Arg Leu Glu Glu Asp Arg Lys Arg Tyr
   465 470 475 480
   Leu Met Thr Leu Leu Asp Asp Ile Lys Gly Ala Asn Asp Leu Ala Lys
   485 490 495
   Phe His Gln Met Leu Met Lys Ile Ile Met Lys
   500 505
<210> 4
   <211> 390
   <212> PRT
   <213> Mumps virus
<220>
   <221> MISC_FEATURE
   <222> (1)..(390)
   <223> Phosphoprotein P (Swissprot P19717)
<400> 4
   Met Asp Gln Phe Ile Lys Gln Asp Glu Thr Gly Asp Leu Ile Glu Thr
   1 5 10 15
   Gly Met Asn Val Ala Asn His Phe Leu Ser Ala Pro Ile Gln Gly Thr
   20 25 30
   Asn Ser Leu Ser Lys Ala Ser Ile Ile Pro Gly Val Ala Pro Val Leu
   35 40 45
   Ile Gly Asn Pro Glu Gln Lys Asn Ile Gln His Pro Thr Ala Ser His
   50 55 60
   Gln Gly Ser Lys Ser Lys Gly Ser Gly Ser Gly Val Arg Ser Ile Ile
   65 70 75 80
   Val Pro Pro Ser Glu Ala Ser Asn Gly Gly Thr Gln Ile Pro Glu Pro
   85 90 95
   Leu Phe Ala Gln Thr Gly Gln Gly Gly Ile Val Thr Thr Val Tyr Gln
                   100                 105                 110
   Asp Pro Thr Ile Gln Pro Thr Gly Ser Tyr Arg Ser Val Glu Leu Ala
           115                 120                 125
   Lys Ile Gly Lys Glu Arg Met Ile Asn Arg Phe Val Glu Lys Pro Arg
       130                 135                 140
   Thr Ser Thr Pro Val Thr Glu Phe Lys Arg Gly Ala Gly Ser Arg Ala
   145                 150                 155                 160
   Gln Gly Gln Thr Ile Gln Glu Glu Gly Ile Asp Gly Asn Gly Ala Ser
                   165                 170                 175
   Ala Gly Ser Lys Glu Arg Ser Gly Ser Leu Ser Gly Ala Thr Leu Tyr
               180                 185                 190
   Ala His Leu Ser Leu Pro Gln Gln Asp Ser Thr Pro Ala Asn Val Gly
           195                 200                 205
   Ile Ala Pro Gln Ser Ala Ile Ser Ala Asn Glu Ile Met Asp Leu Leu
       210                 215                 220
   Arg Gly Met Asp Ala Arg Leu Gln His Leu Glu Gln Lys Val Asp Lys
   225                 230                 235                 240
   Val Leu Ala Gln Gly Ser Met Val Thr Gln Ile Lys Asn Glu Leu Ser
                   245                 250                 255
   Thr Val Lys Thr Thr Leu Ala Thr Ile Glu Gly Met Met Ala Thr Val
               260                 265                 270
   Lys Ile Met Asp Pro Gly Asn Pro Thr Gly Val Pro Val Asp Glu Leu
           275                 280                 285
   Arg Arg Ser Phe Ser Asp His Val Thr Ile Val Ser Gly Pro Gly Asp
       290                 295                 300
   Val Pro Phe Ser Ser Ser Glu Glu Pro Thr Leu Tyr Leu Asp Glu Leu
   305                 310                 315                 320
   Ala Arg Pro Val Ser Lys Pro Arg Pro Ala Lys Gln Thr Lys Pro Gln
                   325                 330                 335
   Pro Val Lys Asp Leu Ala Gly Arg Lys Val Met Ile Thr Lys Met Ile
               340                 345                 350
   Thr Asp Cys Val Ala Asn Pro Gln Met Lys Gln Ala Phe Glu Gln Arg
           355                 360                 365
   Leu Ala Lys Ala Ser Thr Glu Asp Ala Leu Asn Asp Ile Lys Lys Asp
       370                 375                 380
   Ile Ile Arg Ser Ala Ile
   385                 390
<210> 5
   <211> 294
   <212> PRT
   <213> Human Metapneumovirus
<220>
   <221> MISC_FEATURE
   <222> (1)..(517)
   <223> phosphoprotein P (Swissprot Q91KZ5)
<900> 5
<210> 6
   <211> 391
   <212> PRT
   <213> Human respiratory syncytial virus
<220>
   <221> MISC_FEATURE
   <222> (1)..(391)
   <223> nucleocapsid protein (strain LONG)
<400> 6
   Met Ala Leu Ser Lys Val Lys Leu Asn Asp Thr Leu Asn Lys Asp Gln
   1 5 10 15
   Leu Leu Ser Ser Ser Lys Tyr Thr Ile Gln Arg Ser Thr Gly Asp Ser
   20 25 30
   Ile Asp Thr Pro Asn Tyr Asp Val Gln Lys His Ile Asn Lys Leu Cys
   35 40 45
   Gly Met Leu Leu Ile Thr Glu Asp Ala Asn His Lys Phe Thr Gly Leu
   50 55 60
   Ile Gly Met Leu Tyr Ala Met Ser Arg Leu Gly Arg Glu Asp Thr Ile
   65 70 75 80
   Lys Ile Leu Arg Asp Ala Gly Tyr His Val Lys Ala Asn Gly Val Asp
   85 90 95
   Val Thr Thr His Arg Gln Asp Ile Asn Gly Lys Glu Met Lys Phe Glu
   100 105 110
   Val Leu Thr Leu Ser Ser Leu Thr Thr Glu Ile Gln Ile Asn Ile Glu
   115 120 125
   Ile Glu Ser Arg Lys Ser Tyr Lys Lys Met Leu Lys Glu Met Gly Glu
   130 135 140
   Val Ala Pro Glu Tyr Arg His Asp Ser Pro Asp Cys Gly Met Ile Ile
   145 150 155 160
   Leu Cys Ile Ala Ala Leu Val Ile Thr Lys Leu Ala Ala Gly Asp Arg
   165 170 175
   Ser Gly Leu Thr Ala Val Ile Arg Arg Ala Asn Asn Val Leu Lys Asn
   180 185 190
   Glu Met Lys Arg Tyr Lys Gly Leu Leu Pro Lys Asp Ile Ala Asn Ser
   195 200 205
   Phe Tyr Glu Val Phe Glu Lys Tyr Pro His Phe Ile Asp Val Phe Val
   210 215 220
   His Phe Gly Ile Ala Gln Ser Ser Thr Arg Gly Gly Ser Arg Val Glu
   225 230 235 240
   Gly Ile Phe Ala Gly Leu Phe Met Asn Ala Tyr Gly Ala Gly Gln Val
   245 250 255
   Met Leu Arg Trp Gly Val Leu Ala Lys Ser Val Lys Asn Ile Met Leu
               260                 265                 270
   Gly His Ala Ser Val Gln Ala Glu Met Glu Gln Val Val Glu Val Tyr
           275                 280                 285
   Glu Tyr Ala Gln Lys Leu Gly Gly Glu Ala Gly Phe Tyr His Ile Leu
       290                 295                 300
   Asn Asn Pro Lys Ala Ser Leu Leu Ser Leu Thr Gln Phe Pro His Phe
   305                 310                 315                 320
   Ser Ser Val Val Leu Gly Asn Ala Ala Gly Leu Gly Ile Met Gly Glu
                   325                 330                 335
   Tyr Arg Gly Thr Pro Arg Asn Gln Asp Leu Tyr Asp Ala Ala Lys Ala
               340                 345                 350
   Tyr Ala Glu Gln Leu Lys Glu Asn Gly Val Ile Asn Tyr Ser Val Leu
           355                 360                 365
   Asp Leu Thr Ala Glu Glu Leu Glu Ala Ile Lys His Gln Leu Asn Pro
       370                 375                 380
   Lys Asp Asn Asp Val Glu Leu
   385                 390
<210> 7
   <211> 524
   <212> PRT
   <213> Sendai virus
<220>
   <221> MISC_FEATURE
   <222> (1)..(524)
   <223> nucleocapsid protein (Swissprot Q9DUE3)
<400> 7
   Met Ala Gly Leu Leu Ser Thr Phe Asp Thr Phe Ser Ser Arg Arg Ser
   1               5                   10                  15
   Glu Ser Ile Asn Lys Ser Gly Gly Gly Ala Val Ile Pro Gly Gln Arg
               20                  25                  30
   Ser Thr Val Ser Val Phe Val Leu Gly Pro Ser Val Thr Asp Asp Ala
           35                  40                  45
   Asp Lys Leu Phe Ile Ala Thr Thr Phe Leu Ala His Ser Leu Asp Thr
   50 55 60
   Asp Lys Gln His Ser Gln Arg Gly Gly Phe Leu Val Ser Leu Leu Ala
   65 70 75 80
   Met Ala Tyr Ser Ser Pro Glu Leu Tyr Leu Thr Thr Asn Gly Val Asn
   85 90 95
   Ala Asp Val Lys Tyr Val Ile Tyr Asn Ile Glu Lys Asp Pro Lys Arg
   100 105 110
   Thr Lys Thr Asp Gly Phe Ile Val Lys Thr Arg Asp Met Glu Tyr Glu
   115 120 125
   Arg Thr Thr Glu Trp Leu Phe Gly Pro Met Val Asn Lys Ser Pro Leu
   130 135 140
   Phe Gln Gly Gln Arg Asp Ala Ala Asp Pro Asp Thr Leu Leu Gln Ile
   145 150 155 160
   Tyr Gly Tyr Pro Ala Cys Leu Gly Ala Ile Ile Val Gln Val Trp Ile
   165 170 175
   Val Leu Val Lys Ala Ile Thr Ser Ser Ala Gly Leu Arg Lys Gly Phe
   180 185 190
   Phe Asn Arg Leu Glu Ala Phe Arg Gln Asp Gly Thr Val Lys Gly Ala
   195 200 205
   Leu Val Phe Thr Gly Glu Thr Val Glu Gly Ile Gly Ser Val Met Arg
   210 215 220
   Ser Gln Gln Ser Leu Val Ser Leu Met Val Glu Thr Leu Val Thr Met
   225 230 235 240
   Asn Thr Ala Arg Ser Asp Leu Thr Thr Leu Glu Lys Asn Ile Gln Ile
   245 250 255
   Val Gly Asn Tyr Ile Arg Asp Ala Gly Leu Ala Ser Phe Met Asn Thr
   260 265 270
   Ile Lys Tyr Gly Val Glu Thr Lys Met Ala Ala Leu Thr Leu Ser Asn
   275 280 285
   Leu Arg Pro Asp Ile Asn Lys Leu Arg Ser Leu Ile Asp Thr Tyr Leu
   290 295 300
   Ser Lys Gly Pro Arg Ala Pro Phe Ile Cys Ile Leu Lys Asp Pro Val
   305 310 315 320
   His Gly Glu Phe Ala Pro Gly Asn Tyr Pro Ala Leu Trp Ser Tyr Ala
   325 330 335
   Met Gly Val Ala Val Val Gln Asn Lys Ala Met Gln Gln Tyr Val Thr
   340 345 350
   Gly Arg Thr Tyr Leu Asp Met Glu Met Phe Leu Leu Gly Gln Ala Val
   355 360 365
   Ala Lys Asp Ala Glu Ser Lys Ile Ser Ser Ala Leu Glu Asp Glu Leu
   370 375 380
   Gly Val Thr Asp Thr Ala Lys Glu Arg Leu Arg His His Leu Ala Asn
   385 390 395 400
   Leu Ser Gly Gly Asp Gly Ala Tyr His Lys Pro Thr Gly Gly Gly Ala
   405 410 415
   Ile Glu Val Ala Leu Asp Asn Ala Asp Ile Asp Leu Glu Pro Glu Ala
   420 425 430
   His Thr Asp Gln Asp Ala Arg Gly Trp Gly Gly Asp Ser Gly Asp Arg
   435 440 445
   Trp Ala Arg Ser Thr Ser Ser Gly His Phe Ile Thr Leu His Gly Ala
   450 455 460
   Glu Arg Leu Glu Glu Glu Thr Asn Asp Glu Asp Val Ser Asp Ile Glu
   465 470 475 480
   Arg Arg Ile Ala Arg Arg Leu Ala Glu Arg Arg Gln Glu Asp Ala Thr
   485 490 495
   Thr His Glu Asp Glu Gly Arg Asn Asn Gly Val Asp His Asp Glu Glu
   500 505 510
   Asp Asp Ala Ala Ala Ala Ala Gly Met Gly Gly Ile
   515 520
<210> 8
   <211> 525
   <212> PRT
   <213> Measles virus
<220>
   <221> MISC_FEATURE
   <222> (1)..(525)
   <223> Nucleocapsid protein (Swissprot Q89933)
<400> 8
   Met Ala Thr Leu Leu Arg Ser Leu Ala Leu Phe Lys Arg Asn Lys Asp
   1               5                   10                  15
   Lys Pro Pro Ile Thr Ser Gly Ser Gly Gly Ala Ile Arg Gly Ile Lys
               20                  25                  30
   His Ile Ile Ile Val Pro Ile Pro Gly Asp Ser Ser Ile Thr Thr Arg
           35                  40                  45
   Ser Arg Leu Leu Asp Arg Leu Val Arg Leu Ile Gly Asn Pro Asp Val
       50                  55                  60
   Ser Gly Pro Lys Leu Thr Gly Ala Leu Ile Gly Ile Leu Ser Leu Phe
   65                  70                  75                  80
   Val Glu Ser Pro Gly Gln Leu Ile Gln Arg Ile Thr Asp Asp Pro Asp
                   85                  90                  95
   Val Ser Ile Arg Leu Leu Glu Val Val Gln Ser Asp Gln Ser Gln Ser
               100                 105                 110
   Gly Leu Thr Phe Ala Ser Arg Gly Thr Asn Met Glu Asp Glu Ala Asp
           115                 120                 125
   Gln Tyr Phe Ser His Asp Asp Pro Ile Ser Ser Asp Gln Ser Arg Phe
       130                 135                 140
   Gly Trp Phe Glu Asn Lys Glu Ile Ser Asp Ile Glu Val Gln Asp Pro
   145                 150                 155                 160
   Glu Gly Phe Asn Met Ile Leu Gly Thr Ile Leu Ala Gln Ile Trp Val
                   165                 170                 175
   Leu Leu Ala Lys Ala Val Thr Ala Pro Asp Thr Ala Ala Asp Ser Glu
               180                 185                 190
   Leu Arg Arg Trp Ile Lys Tyr Thr Gln Gln Arg Arg Val Val Gly Glu
           195                 200                 205
   Phe Arg Leu Glu Arg Lys Trp Leu Asp Val Val Arg Asn Arg Ile Ala
       210                 215                 220
   Glu Asp Leu Ser Leu Arg Arg Phe Met Val Ala Leu Ile Leu Asp Ile
   225                 230                 235                 240
   Lys Arg Thr Pro Gly Asn Lys Pro Arg Ile Ala Glu Met Ile Cys Asp
                   245                 250                 255
   Ile Asp Thr Tyr Ile Val Glu Ala Gly Leu Ala Ser Phe Ile Leu Thr
               260                 265                 270
   Ile Lys Phe Gly Ile Glu Thr Met Tyr Pro Ala Leu Gly Leu His Glu
           275                 280                 285
   Phe Ala Gly Glu Leu Ser Thr Leu Glu Ser Leu Met Asn Leu Tyr Gln
       290                 295                 300
   Gln Met Gly Glu Thr Ala Pro Tyr Met Val Ile Leu Glu Asn Ser Ile
   305                 310                 315                 320
   Gln Asn Lys Phe Ser Ala Gly Ser Tyr Pro Leu Leu Trp Ser Tyr Ala
                   325                 330                 335
   Met Gly Val Gly Val Glu Leu Glu Asn Ser Met Gly Gly Leu Asn Phe
               340                 345                 350
   Gly Arg Ser Tyr Phe Asp Pro Ala Tyr Phe Arg Leu Gly Gln Glu Met
           355                 360                 365
   Val Arg Arg Ser Ala Gly Lys Val Ser Ser Thr Leu Ala Ser Glu Leu
       370                 375                 380
   Gly Ile Thr Ala Glu Asp Ala Arg Leu Val Ser Glu Ile Ala Met His
   385                 390                 395                 400
   Thr Thr Glu Asp Lys Ile Ser Arg Ala Val Gly Pro Arg Gln Ala Gln
                   405                 410                 415
   Val Ser Phe Leu His Gly Asp Gln Ser Glu Asn Glu Leu Pro Arg Leu
               420                 425                 430
   Gly Gly Lys Glu Asp Arg Arg Val Lys Gln Ser Arg Gly Glu Ala Arg
           435                 440                 445
   Glu Ser Tyr Arg Glu Thr Gly Pro Ser Arg Ala Ser Asp Ala Arg Ala
   450 455 460
   Ala His Leu Pro Thr Gly Thr Pro Leu Asp Ile Asp Thr Ala Ser Glu
   465 470 475 480
   Ser Ser Gln Asp Pro Gln Asp Ser Arg Arg Ser Ala Asp Ala Leu Leu
   485 490 495
   Arg Leu Gln Ala Met Ala Gly Ile Ser Glu Glu Gln Gly Ser Asp Thr
   500 505 510
   Asp Thr Pro Ile Val Tyr Asn Asp Arg Asn Leu Leu Asp
   515 520 525
<210> 9
   <211> 553
   <212> PRT
   <213> Mumps virus
<220>
   <221> MISC_FEATURE
   <222> (1)..(553)
   <223> Nucleocapsid protein (Swissprot P21277)
<400> 9
   Met Ser Ser Val Leu Lys Ala Phe Glu Arg Phe Thr Ile Glu Gln Glu
   1 5 10 15
   Leu Gln Asp Arg Gly Glu Glu Gly Ser Ile Pro Pro Glu Thr Leu Lys
   20 25 30
   Ser Ala Val Lys Val Phe Val Ile Asn Thr Pro Asn Pro Thr Thr Arg
   35 40 45
   Tyr Gln Met Leu Asn Phe Cys Leu Arg Ile Ile Cys Ser Gln Asn Arg
   50 55 60
   Arg Ala Ser His Arg Val Gly Ala Leu Ile Ala Leu Phe Ser Leu Pro
   65 70 75 80
   Ser Ala Gly Met Gln Asn His Ile Arg Leu Ala Asp Arg Ser Pro Glu
   85 90 95
   Ala Gln Ile Glu Arg Cys Glu Ile Asp Gly Phe Glu Pro Gly Thr Tyr
   100 105 110
   Arg Leu Ile Pro Asn Ala Arg Ala Asn Leu Thr Ala Asn Glu Ile Ala
           115                 120                 125
   Ala Tyr Ala Leu Leu Ala Asp Asp Leu Pro Pro Thr Ile Asn Asn Gly
       130                 135                 140
   Thr Pro Tyr Val His Ala Asp Val Glu Leu Gln Pro Cys Asp Glu Ile
   145                 150                 155                 160
   Glu Gln Phe Leu Asp Arg Cys Tyr Ser Val Leu Ile Gln Ala Trp Val
                   165                 170                 175
   Met Val Cys Lys Cys Met Thr Ala Tyr Asp Gln Pro Ala Gly Ser Ala
               180                 185                 190
   Asp Arg Arg Phe Ala Lys Tyr Gln Gln Gln Gly Arg Leu Glu Ala Arg
           195                 200                 205
   Tyr Met Leu Gln Pro Glu Ala Gln Arg Leu Ile Gln Thr Ala Ile Arg
       210                 215                 220
   Lys Ser Leu Val Val Arg Gln Tyr Leu Thr Phe Glu Leu Gln Leu Ala
   225                 230                 235                 240
   Arg Arg Gln Gly Leu Leu Ser Asn Arg Tyr Tyr Ala Met Val Gly Asp
                   245                 250                 255
   Ile Gly Lys Tyr Ile Glu Asn Ser Gly Leu Thr Ala Phe Phe Leu Thr
               260                 265                 270
   Leu Lys Tyr Ala Leu Gly Thr Lys Trp Ser Pro Leu Ser Leu Ala Ala
           275                 280                 285
   Phe Thr Gly Glu Leu Thr Lys Leu Arg Ser Leu Met Met Leu Tyr Arg
       290                 295                 300
   Asp Ile Gly Glu Gln Ala Arg Tyr Leu Ala Leu Leu Glu Ala Pro Gln
   305                 310                 315                 320
   Ile Met Asp Phe Ala Pro Gly Gly Tyr Pro Leu Ile Phe Ser Tyr Ala
                   325                 330                 335
   Met Gly Val Gly Ser Val Leu Asp Val Gln Met Arg Asn Tyr Thr Tyr
               340                 345                 350
   Ala Arg Pro Phe Leu Asn Gly Tyr Tyr Phe Gln Ile Gly Val Glu Thr
           355                 360                 365
   Ala Arg Arg Gln Gln Gly Thr Val Asp Asn Arg Val Ala Asp Asp Leu
       370                 375                 380
   Gly Leu Thr Pro Glu Gln Arg Asn Glu Val Thr Gln Leu Val Asp Arg
   385                 390                 395                 400
   Leu Ala Arg Gly Arg Gly Ala Gly Ile Pro Gly Gly Pro Val Asn Pro
                   405                 410                 415
   Phe Val Pro Pro Val Gln Gln Gln Gln Pro Ala Ala Val Tyr Ala Asp
               420                 425                 430
   Ile Pro Ala Leu Glu Glu Ser Asp Asp Asp Gly Asp Glu Asp Gly Gly
           435                 440                 445
   Ala Gly Phe Gln Asn Gly Val Gln Val Pro Ala Val Arg Gln Gly Gly
       450                 455                 460
   Gln Thr Asp Phe Arg Ala Gln Pro Leu Gln Asp Pro Ile Gln Ala Gln
   465                 470                 475                 480
   Leu Phe Met Pro Leu Tyr Pro Gln Val Ser Asn Ile Pro Asn Asn Arg
                   485                 490                 495
   Ile Ile Arg Ser Ile Ala Ser Gly Gly Trp Lys Thr Lys Ile Tyr Tyr
               500                 505                 510
   Asp Thr Thr Arg Met Val Ile Leu Asn Lys Met Gln Gly Ala Asn Thr
           515                 520                 525
   Glu Thr Leu Ser Gln Thr Ile Pro Ile Lys Thr His Ser Cys Lys Trp
       530                 535                 540
   Ala Thr Gly Met Ser Lys Ser Leu Thr
   545                 550
<210> 10
   <211> 394
   <212> PRT
   <213> Human Metapneumovirus
<220>
   <221> MISC_FEATURE
   <222> (1)..(394)
   <223> nucleocapsid protein N (Swissprot Q91F57)
<400> 10
   Met Ser Leu Gln Gly Ile His Leu Ser Asp Leu Ser Tyr Lys His Ala
   1 5 10 15
   Ile Leu Lys Glu Ser Gln Tyr Thr Ile Lys Arg Asp Val Gly Thr Thr
   20 25 30
   Thr Ala Val Thr Pro Ser Ser Leu Gln Gln Glu Ile Thr Leu Leu Cys
   35 40 45
   Gly Glu Ile Leu Tyr Ala Lys His Ala Asp Tyr Lys Tyr Ala Ala Glu
   50 55 60
   Ile Gly Ile Gln Tyr Ile Ser Thr Ala Leu Gly Ser Glu Arg Val Gln
   65 70 75 80
   Gln Ile Leu Arg Asn Ser Gly Ser Glu Val Gln Val Val Leu Thr Arg
   85 90 95
   Thr Tyr Ser Leu Gly Lys Ile Lys Asn Asn Lys Gly Glu Asp Leu Gln
   100 105 110
   Met Leu Asp Ile His Gly Val Glu Lys Ser Trp Val Glu Glu Ile Asp
   115 120 125
   Lys Glu Ala Arg Lys Thr Met Ala Thr Leu Leu Lys Glu Ser Ser Gly
   130 135 140
   Asn Ile Pro Gln Asn Gln Arg Pro Ser Ala Pro Asp Thr Pro Ile Ile
   145 150 155 160
   Leu Leu Cys Val Gly Ala Leu Ile Phe Thr Lys Leu Ala Ser Thr Ile
   165 170 175
   Glu Val Gly Leu Glu Thr Thr Val Arg Arg Ala Asn Arg Val Leu Ser
   180 185 190
   Asp Ala Leu Lys Arg Tyr Pro Arg Met Asp Ile Pro Lys Ile Ala Arg
   195 200 205
   Ser Phe Tyr Asp Leu Phe Glu Gln Lys Val Tyr His Arg Ser Leu Phe
   210 215 220
   Ile Glu Tyr Gly Lys Ala Leu Gly Ser Ser Ser Thr Gly Ser Lys Ala
   225 230 235 240
   Glu Ser Leu Phe Val Asn Ile Phe Met Gln Ala Tyr Gly Ala Gly Gln
   245 250 255
   Thr Met Leu Arg Trp Gly Val Ile Ala Arg Ser Ser Asn Asn Ile Met
   260 265 270
   Leu Gly His Val Ser Val Gln Ala Glu Leu Lys Gln Val Thr Glu Val
   275 280 285
   Tyr Asp Leu Val Arg Glu Met Gly Pro Glu Ser Gly Leu Leu His Leu
   290 295 300
   Arg Gln Ser Pro Lys Ala Gly Leu Leu Ser Leu Ala Asn Cys Pro Asn
   305 310 315 320
   Phe Ala Ser Val Val Leu Gly Asn Ala Ser Gly Leu Gly Ile Ile Gly
   325 330 335
   Met Tyr Arg Gly Arg Val Pro Asn Thr Glu Leu Phe Ser Ala Ala Glu
   340 345 350
   Ser Tyr Ala Lys Ser Leu Lys Glu Ser Asn Lys Ile Asn Phe Ser Ser
   355 360 365
   Leu Gly Leu Thr Asp Glu Glu Lys Glu Ala Ala Glu His Phe Leu Asn
   370 375 380
   Val Ser Asp Asp Ser Gln Asn Asp Tyr Glu
   385 390
<210> 11
   <211> 31
   <212> DNA
   <213> Artificial sequence
<220>
   <223> primer
<400> 11
   gagggatcca tcatggaaaa gtttgctcct g 31
<210> 12
   <211> 27
   <212> DNA
   <213> Artificial sequence
<220>
   <223> primer
<400> 12
   ctgttggtgt tgtgtgttga agtgcag 27
<210> 13
   <211> 31
   <212> DNA
   <213> Artificial sequence
<220>
   <223> primer
<400> 13
   gagggatcct ctgctaggga tggtataaga g 31
<210> 14
   <211> 36
   <212> DNA
   <213> Artificial sequence
<220>
   <223> primer
<400> 14
   gagggatcca aaatcagaac tgaagcatta atgacc 36
<210> 15
   <211> 34
   <212> DNA
   <213> Artificial sequence
<220>
   <223> primer
<400> 15
   gagggatccg aggaaagtga aaagatggca aaag 34
<210> 16
   <211> 31
   <212> DNA
   <213> Artificial sequence
<220>
   <223> primer
<400> 16
   gagggatccg agaaattgaa caacctgttg g 31
<210> 17
   <211> 44
   <212> DNA
   <213> Artificial sequence
<220>
   <223> primer
<400> 17
   gatccaatga tagtgacaat gatctatcac ttgaagattt ctga 44
<210> 18
   <211> 40
   <212> DNA
   <213> Artificial sequence
<220>
   <223> primer
<400> 18
   tcagaaatct tcaagtgata gatcattgtc actatcattg 40
<210> 19
   <211> 33
   <212> DNA
   <213> Artificial sequence
<220>
   <223> primer
<400> 19
   gagggatcca tggctcttag caaagtcaag ttg 33
<210> 20
   <211> 32
   <212> DNA
   <213> Artificial sequence
<220>
   <223> primer
<400> 20
   ttaactcaaa gctctacatc attatctttt gg 32
<210> 21
   <211> 41
   <212> DNA
   <213> Artificial sequence
<220>
   <223> primer
<400> 21
   gatccgatag tgacaatgat ctatcacttg aagatttctg a 41
<210> 22
   <211> 37
   <212> DNA
   <213> Artificial sequence
<220>
   <223> primer
<400> 22
   t cagaaatct tcaagtgata gatcattgtc actatcg 37
<210> 23
   <211> 241
   <212> PRT
   <213> Bovine respiratory syncytial virus
<220>
   <221> MISC_FEATURE
   <222> (1)..(241)
   <223> phopshoprotein P (strain A51908, Swissprot P33454)
<400> 23
   Met Glu Lys Phe Ala Pro Glu Phe His Gly Glu Asp Ala Asn Thr Lys
   1               5                   10                  15
   Ala Thr Lys Phe Leu Glu Ser Leu Lys Gly Lys Phe Thr Ser Ser Lys
               20                  25                  30
   Asp Ser Arg Lys Lys Asp Ser Ile Ile Ser Val Asn Ser Ile Asp Ile
           35                  40                  45
   Glu Leu Pro Lys Glu Ser Pro Ile Thr Ser Thr Asn His Asn Ile Asn
       50                  55                  60
   Gln Pro Ser Glu Ile Asn Asp Thr Ile Ala Ala Asn Gln Val His Ile
   65                  70                  75                  80
   Arg Lys Pro Leu Val Ser Phe Lys Glu Glu Leu Pro Ser Ser Glu Asn
                   85                  90                  95
   Pro Phe Thr Lys Leu Tyr Lys Glu Thr Ile Glu Thr Phe Asp Asn Asn
               100                 105                 110
   Glu Glu Glu Ser Ser Tyr Ser Tyr Asp Glu Ile Asn Asp Gln Thr Asn
           115                 120                 125
   Asp Asn Ile Thr Ala Arg Leu Asp Arg Ile Asp Glu Lys Leu Ser Glu
       130                 135                 140
   Ile Ile Gly Met Leu His Thr Leu Val Val Ala Ser Ala Gly Pro Thr
   145                 150                 155                 160
   Ala Ala Arg Asp Gly Ile Arg Asp Ala Met Val Gly Leu Arg Glu Glu
                   165                 170                 175
   Met Ile Glu Lys Ile Arg Ser Glu Ala Leu Met Thr Asn Asp Arg Leu
   180 185 190
   Glu Ala Met Ala Arg Leu Arg Asp Glu Glu Ser Glu Lys Met Thr Lys
   195 200 205
   Asp Thr Ser Asp Glu Val Lys Leu Thr Pro Thr Ser Glu Lys Leu Asn
   210 215 220
   Met Val Leu Glu Asp Glu Ser Ser Asp Asn Asp Leu Ser Leu Glu Asp
   225 230 235 240
   Phe
<210> 24
   <211> 391
   <212> PRT
   <213> Bovine respiratory syncytial virus
<220>
   <221> MISC_FEATURE
   <222> (1)..(391)
   <223> nucleocapsid protein N (strain 391-2, Swissprot P35943)
<400> 24
   Met Ala Leu Ser Lys Val Lys Leu Asn Asp Thr Phe Asn Lys Asp Gln
   1 5 10 15
   Leu Leu Ser Thr Ser Lys Tyr Thr Ile Gln Arg Ser Thr Gly Asp Asn
   20 25 30
   Ile Asp Ile Pro Asn Tyr Asp Val Gln Lys His Leu Asn Lys Leu Cys
   35 40 45
   Gly Met Leu Leu Ile Thr Glu Asp Ala Asn His Lys Phe Thr Gly Leu
   50 55 60
   Ile Gly Met Leu Tyr Ala Met Ser Arg Leu Gly Arg Glu Asp Thr Leu
   65 70 75 80
   Lys Ile Leu Lys Asp Ala Gly Tyr Gln Val Arg Ala Asn Gly Val Asp
   85 90 95
   Val Ile Thr His Arg Gln Asp Val Asn Gly Lys Glu Met Lys Phe Glu
   100 105 110
   Val Leu Thr Leu Val Ser Leu Thr Ser Glu Val Gln Gly Asn Ile Glu
   115 120 125
   Ile Glu Ser Arg Lys Ser Tyr Lys Lys Met Leu Lys Glu Met Gly Glu
   130 135 140
   Val Ala Pro Glu Tyr Arg His Asp Phe Pro Asp Cys Gly Met Ile Val
   145 150 155 160
   Leu Cys Val Ala Ala Leu Val Ile Thr Lys Leu Ala Ala Gly Asp Arg
   165 170 175
   Ser Gly Leu Thr Ala Val Ile Arg Arg Ala Asn Asn Val Leu Arg Asn
   180 185 190
   Glu Met Lys Arg Tyr Lys Gly Leu Ile Pro Lys Asp Ile Ala Asn Ser
   195 200 205
   Phe Tyr Glu Val Phe Glu Lys Tyr Pro His Tyr Ile Asp Val Phe Val
   210 215 220
   His Phe Gly Ile Ala Gln Ser Ser Thr Arg Gly Gly Ser Arg Val Glu
   225 230 235 240
   Gly Ile Phe Ala Gly Leu Phe Met Asn Ala Tyr Gly Ala Gly Gln Val
   245 250 255
   Met Leu Arg Trp Gly Val Leu Ala Lys Ser Val Lys Asn Ile Met Leu
   260 265 270
   Gly His Ala Ser Val Gln Ala Glu Met Glu Gln Val Val Glu Val Tyr
   275 280 285
   Glu Tyr Ala Gln Lys Leu Gly Gly Glu Ala Gly Phe Tyr His Ile Leu
   290 295 300
   Asn Asn Pro Lys Ala Ser Leu Leu Ser Leu Thr Gln Phe Pro Asn Phe
   305 310 315 320
   Ser Ser Val Val Leu Gly Asn Ala Ala Gly Leu Gly Ile Met Gly Glu
   325 330 335
   Tyr Arg Gly Thr Pro Arg Asn Gln Asp Leu Tyr Asp Ala Ala Lys Ala
   340 345 350
   Tyr Ala Glu Gln Leu Lys Glu Asn Gly Val Ile Asn Tyr Ser Val Leu
   355 360 365
   Asp Leu Thr Thr Glu Glu Leu Glu Ala Ile Lys Asn Gln Leu Asn Pro
   370 375 380
   Lys Asp Asn Asp Val Glu Leu
   385 390
<210> 25
   <211> 294
   <212> PRT
   <213> Human metapneumovirus
<220>
   <221> MISC_FEATURE
   <222> (1)..(294)
   <223> phosphoprotein P (Swissprot Q91KZ5)
<400> 25
   Met Ser Phe Pro Glu Gly Lys Asp Ile Leu Phe Met Gly Asn Glu Ala
   1 5 10 15
   Ala Lys Leu Ala Glu Ala Phe Gln Lys Ser Leu Arg Lys Pro Gly His
   20 25 30
   Lys Arg Ser Gln Ser Ile Ile Gly Glu Lys Val Asn Thr Val Ser Glu
   35 40 45
   Thr Leu Glu Leu Pro Thr Ile Ser Arg Pro Ala Lys Pro Thr Ile Pro
   50 55 60
   Ser Glu Pro Lys Leu Ala Trp Thr Asp Lys Gly Gly Ala Thr Lys Thr
   65 70 75 80
   Glu Ile Lys Gln Ala Ile Lys Val Met Asp Pro Ile Glu Glu Glu Glu
   85 90 95
   Ser Thr Glu Lys Lys Val Leu Pro Ser Ser Asp Gly Lys Thr Pro Ala
   100 105 110
   Glu Lys Lys Leu Lys Pro Ser Thr Asn Thr Lys Lys Lys Val Ser Phe
   115 120 125
   Thr Pro Asn Glu Pro Gly Lys Tyr Thr Lys Leu Glu Lys Asp Ala Leu
   130 135 140
   Asp Leu Leu Ser Asp Asn Glu Glu Glu Asp Ala Glu Ser Ser Ile Leu
   145 150 155 160
   Thr Phe Glu Glu Arg Asp Thr Ser Ser Leu Ser Ile Glu Ala Arg Leu
   165 170 175
   Glu Ser Ile Glu Glu Lys Leu Ser Met Ile Leu Gly Leu Leu Arg Thr
   180 185 190
   Leu Asn Ile Ala Thr Ala Gly Pro Thr Ala Ala Arg Asp Gly Ile Arg
   195 200 205
   Asp Ala Met Ile Gly Val Arg Glu Glu Leu Ile Ala Asp Ile Ile Lys
   210 215 220
   Glu Ala Lys Gly Lys Ala Ala Glu Met Met Glu Glu Glu Met Ser Gln
   225 230 235 240
   Arg Ser Lys Ile Gly Asn Gly Ser Val Lys Leu Thr Glu Lys Ala Lys
   245 250 255
   Glu Leu Asn Lys Ile Val Glu Asp Glu Ser Thr Ser Gly Glu Ser Glu
   260 265 270
   Glu Glu Glu Glu Pro Lys Asp Thr Gln Asp Asn Ser Gln Glu Asp Asp
   275 280 285
   Ile Tyr Gln Leu Ile Met
   290
<210> 26
   <211> 394
   <212> PRT
   <213> Human metapneumovirus
<220>
   <221> MISC_FEATURE
   <222> (1)..(394)
   <223> nucleocapsid protein N (Swissprot Q91F57)
<400> 26
   Met Ser Leu Gln Gly Ile His Leu Ser Asp Leu Ser Tyr Lys His Ala
   1 5 10 15
   Ile Leu Lys Glu Ser Gln Tyr Thr Ile Lys Arg Asp Val Gly Thr Thr
   20 25 30
   Thr Ala Val Thr Pro Ser Ser Leu Gln Gln Glu Ile Thr Leu Leu Cys
               35                  40                  45
   Gly Glu Ile Leu Tyr Ala Lys His Ala Asp Tyr Lys Tyr Ala Ala Glu
       50                  55                  60
   Ile Gly Ile Gln Tyr Ile Ser Thr Ala Leu Gly Ser Glu Arg Val Gln
   65                  70                  75                  80
   Gln Ile Leu Arg Asn Ser Gly Ser Glu Val Gln Val Val Leu Thr Arg
                   85                  90                  95
   Thr Tyr Ser Leu Gly Lys Ile Lys Asn Asn Lys Gly Glu Asp Leu Gln
               100                 105                 110
   Met Leu Asp Ile His Gly Val Glu Lys Ser Trp Val Glu Glu Ile Asp
           115                 120                 125
   Lys Glu Ala Arg Lys Thr Met Ala Thr Leu Leu Lys Glu Ser Ser Gly
       130                 135                 140
   Asn Ile Pro Gln Asn Gln Arg Pro Ser Ala Pro Asp Thr Pro Ile Ile
   145                 150                 155                 160
   Leu Leu Cys Val Gly Ala Leu Ile Phe Thr Lys Leu Ala Ser Thr Ile
                   165                 170                 175
   Glu Val Gly Leu Glu Thr Thr Val Arg Arg Ala Asn Arg Val Leu Ser
               180                 185                 190
   Asp Ala Leu Lys Arg Tyr Pro Arg Met Asp Ile Pro Lys Ile Ala Arg
           195                 200                 205
   Ser Phe Tyr Asp Leu Phe Glu Gln Lys Val Tyr His Arg Ser Leu Phe
       210                 215                 220
   Ile Glu Tyr Gly Lys Ala Leu Gly Ser Ser Ser Thr Gly Ser Lys Ala
   225                 230                 235                 240
   Glu Ser Leu Phe Val Asn Ile Phe Met Gln Ala Tyr Gly Ala Gly Gln
                   245                 250                 255
   Thr Met Leu Arg Trp Gly Val Ile Ala Arg Ser Ser Asn Asn Ile Met
               260                 265                 270
   Leu Gly His Val Ser Val Gln Ala Glu Leu Lys Gln Val Thr Glu Val
           275                 280                 285
   Tyr Asp Leu Val Arg Glu Met Gly Pro Glu Ser Gly Leu Leu His Leu
       290                 295                 300
   Arg Gln Ser Pro Lys Ala Gly Leu Leu Ser Leu Ala Asn Cys Pro Asn
   305                 310                 315                 320
   Phe Ala Ser Val Val Leu Gly Asn Ala Ser Gly Leu Gly Ile Ile Gly
                   325                 330                 335
   Met Tyr Arg Gly Arg Val Pro Asn Thr Glu Leu Phe Ser Ala Ala Glu
               340                 345                 350
   Ser Tyr Ala Lys Ser Leu Lys Glu Ser Asn Lys Ile Asn Phe Ser Ser
           355                 360                 365
   Leu Gly Leu Thr Asp Glu Glu Lys Glu Ala Ala Glu His Phe Leu Asn
       370                 375                 380
   Val Ser Asp Asp Ser Gln Asn Asp Tyr Glu
   385                 390

## Description

L'invention concerne un procédé de préparation d'un complexe soluble protéine N - protéine P tronquée d'un virus de la famille des *Paramyxoviridae,* les complexes ainsi préparés, ainsi que les protéines N solubles susceptibles d'être isolées à partir de ces complexes. L'invention concerne également des compositions vaccinales comprenant ces complexes protéine N - protéine P tronquée ou des protéines N de *Paramyxoviridae.* De manière préférentielle, la protéine P tronquée est un fragment C-terminal de la protéine P.

En France, comme dans la plupart des pays, le virus respiratoire syncytial (VRS) bovin est le principal agent responsable de maladies respiratoires graves chez le veau (bronchiolites, pneumonies) dans plus de 70 % des exploitations et chez environ 70% des veaux au cours de la première année (Perrin et al., 1979 ; Ames , 1993 ; Elvander, 1996). La mortalité peut atteindre 20% (Wellemans, 1992). C'est un véritable fléau pour les éleveurs qui ne peuvent qu'assister impuissants chaque année, en hiver, à l'apparition de cette maladie de façon systématique. Il y a donc une forte demande des éleveurs à ce sujet pour la mise au point d'une prophylaxie efficace. Des vaccins sont commercialisés, mais ils sont en réalité peu ou pas efficaces.

Il existe la même maladie chez l'homme : c'est l'agent responsable de la bronchiolite du nouveau-né (voir revue Virologie Vol.7 numéro spécial octobre 2003 : infections à virus respiratoire syncytial en pédiatrie). Les données concernant la maladie humaine sont plus précises. Comme pour les bovins, en France, chez l'homme, c'est une maladie épidémique d'hiver. Pendant la première année de la vie, 70% des nourrissons sont infectés par le VRS, et 100% au cours des deux premières années. Ceci représente 500 000 enfants atteints chaque année en France. Une maladie des voies respiratoires basses (bronchiolite) survient chez 20% des nourrissons atteints, et le taux d'hospitalisation varie de 2 à 5% en France, pour une durée de 8 à 9 jours, de 10 à 25% chez les prématurés, 14 à 45% en cas de dysplasie pulmonaire, 15 à 25% en cas d'anomalie cardiaque congénitale. Environ 10% des patients hospitalisés doivent être traités dans une unité de soins intensifs. La mortalité avoisine 0,1 %. Selon un récent rapport de l'Organisation Mondiale de la Santé, il a été estimé que 64 millions de personnes sont infectées chaque année dans le monde, et 160 000 décès seraient dus au VRS. Chaque année aux Etats-Unis, on compte de 18000 à 75000 hospitalisations et près de 2000 morts directement et 17000 indirectement (Magon and Barik, 2004). Le VRS serait également responsable d'un tiers des symptômes grippaux chez l'adulte. En réalité il a été estimé le VRS tuerait quatre fois plus les personnes âgées que les enfants. En tout, le VRS emporterait de 3 à 5 millions de vies par an. De plus, cette maladie semble augmenter les risques de développer de l'asthme chez l'adulte lorsqu'elle est contractée au plus jeune âge (avant 4 mois), par des mécanismes immunologiques complexes encore mal compris.

Même si les VRS humain et bovin sont deux virus distincts, ils sont très proches que ce soit sur le plan structural, moléculaire, antigénique, la maladie qu'ils induisent (tableau clinique, saisonnalité des infections, infection du jeune), et par le fait qu'il n'existe pas de vaccin efficace ni d'antiviraux, en dehors d'un anticorps monoclonal extrêmement coûteux et donc rarement utilisé et d'un antiviral peu efficace et assez toxique qui en réalité déplète les cellules en ATP.

Dans les années soixante, des essais de vaccination ont été réalisés aux Etats-Unis sur des jeunes enfants avec du virus fixé au formol. Les résultats furent catastrophiques, les enfants vaccinés ayant développé des symptômes exacerbés lors de l'infection naturelle par le virus quelques mois plus tard, et entraînant même la mort de plusieurs d'entre eux.

Suite à ces essais malheureux, il n'existe toujours pas de vaccin contre cette maladie pour l'homme, et les vaccins commercialisés pour les bovins sont d'une efficacité plus que douteuse, même s'il n'y a pas d'étude sérieuse en France faite sur ce sujet. Plusieurs vaccins ont cependant été retirés du commerce après des accidents post-vaccinaux chez des bovins.

La difficulté de mettre au point un vaccin contre le VRS peut s'expliquer par les raisons suivantes :
(1) l'infection naturelle ne confère pas une immunité protectrice contre une réinfection, car la mémoire immunitaire locale contre le VRS est de courte durée ;
(2) l'induction d'une immunité cellulaire et humorale forte est associée avec une augmentation de la sévérité de la maladie (sensibilisation par la vaccination) ;
(3) Il n'existe pas d'adjuvant ou de stratégie vaccinale permettant de vacciner efficacement par voie mucosale ;
(4) Les nouveau-nés (moins de deux mois) sont la cible principale de la vaccination, or leur système immunitaire répond mal aux vaccins classiques ;
(5) La présence d'anticorps maternels dans le sang qui n'empêchent pas l'infection ;
(6) La variabilité des souches virales circulantes.

Des vaccins sous-unitaires sont en cours de développement, pour certains sur la base de la protéine de surface G. De nouveaux types de vaccins obtenus par génétique inverse, (c'est-à-dire par atténuation de souche par modification génétique) sont en développement depuis de nombreuses années, pour le VRSH comme pour le VRSB. Pour l'instant aucune utilisation de souche atténuée n'a été validée pour l'homme. Ceci est probablement dû au fait que les souches atténuées déclenchent une réponse immunitaire faible.

Des vaccins ADN basés sur les protéines de membrane F et G du VRSH ou du VRSB sont en cours de développement y compris chez le veau (Taylor et al. 2005, Martinez et al. 1999). Ce type de vaccin confère une immunité protectrice associée pour certaines études à une immunopathologie. L'efficacité de ces vaccins reste à améliorer.

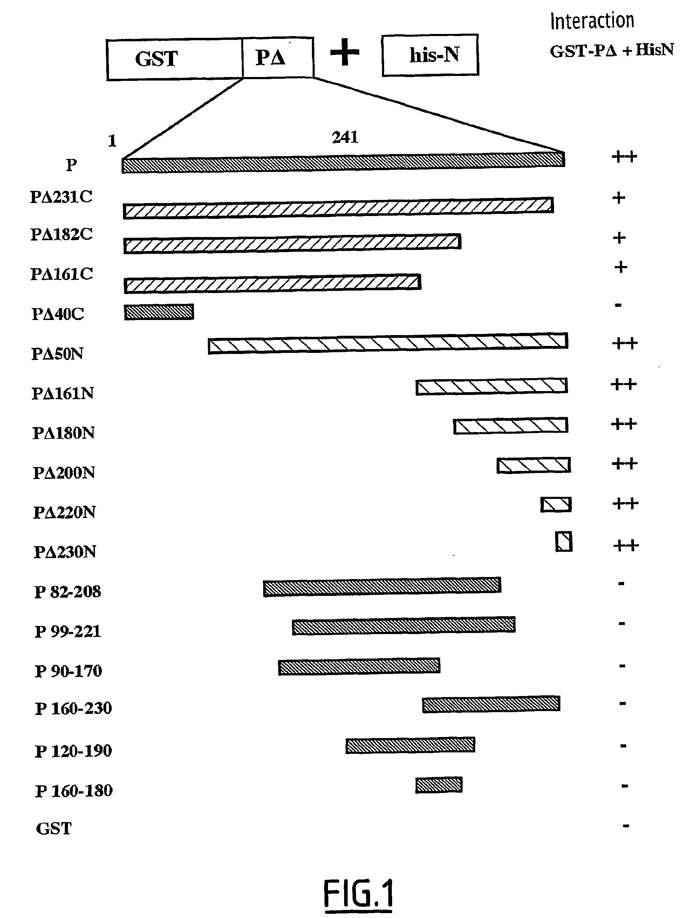

Le VRS est un virus enveloppé possédant un génome à ARN simple brin de polarité négative. Cet ARN code pour 11 protéines et est encapsidé par la protéine de nucléocapside (N) et associé au complexe polymérase composé de deux protéines, L (large) ou polymérase et le co-facteur P (phosphoprotéine) (Figure 1). Cette molécule joue un rôle essentiel dans le fonctionnement de la polymérase : P permet à L de reconnaître l'ARN génomique. Il existe en plus deux co-facteurs pour le VRS, M2-1 et M2-2 qui jouent un rôle de processivité (M2-1) et de régulation de la balance transcription/réplication (M2-2).

Sur la surface de la particule virale on trouve deux protéines majeures, F et G. La protéine F permet la fusion de l'enveloppe virale avec les membranes cellulaires et est impliquée dans la formation de syncytia. La protéine G permet l'attachement du virus à la surface cellulaire. La protéine M (matrice) sert d'intermédiaire entre l'enveloppe virale et le complexe Polymérase/génome. Les deux protéines de surface F et G sont les protéines immunogènes majeures du VRS car elles sont les cibles des anticorps neutralisants.

Cependant les études réalisées chez la souris ont montré que la protéine G est impliquée dans l'induction des réponses vaccinales immunopathologiques de type Th2 (production d'IL-4 et d'IL-5 et recrutement d'éosinophiles) (Sparer et al. 1998). Par contre chez le bovin, aucune de ces deux protéines n'a été associée à des réponses immunopathologiques et les deux permettent l'établissement de réponses protectrices (Taylor et al. 1997).

A l'heure actuelle les recherches de nouveaux candidats vaccins restent majoritairement orientées sur les protéines F et G.

Cependant chez l'homme et le bovin, l'immunité à médiation cellulaire et en particulier la réponse T cytotoxique est une composante cruciale de la protection contre le VRS. Hors chez l'homme comme chez le bovin, la protéine N est le support principal des réponses T cytotoxiques (Goulder et al., 2000). Le veau est un modèle pertinent pour la vaccination contre le VRS. Il a été montré que l'utilisation de vaccine recombinante exprimant la protéine N du VRS générerait une réponse de type cellulaire (Th1) permettant de rééquilibrer la réponse immunitaire (Taylor et al., 1997 ; Gaddum et al., 2003). L'ensemble des travaux actuels plaident donc en faveur d'un vaccin anti-VRS constitué par l'association de plusieurs protéines, en particulier des protéines de surface F et G et de la protéine interne N.

La protéine N est la protéine la plus exprimée dans la cellule infectée et une des plus importantes sur le plan quantitatif dans les particules virales (Collins et al., 2001). Elle entoure le génome viral constitué d'un ARN simple brin, formant des structures hélicoïdales de grande taille. Lorsqu'elle est exprimée seule sous forme recombinante, la protéine N polymérise sur les ARN cellulaires de façon non spécifique. Elle forme alors des structures hélicoïdales (ARN/N) très grosses, insolubles et non purifiables, ressemblant aux nucléocapsides observées dans les cellules infectées (Méric et al., 1994; Bhella et al., 2002).

Cette protéine N est capable d'interagir avec la protéine P du VRS, co-facteur de la polymérase virale L. Des études de cartographie des domaines d'interaction utilisant essentiellement le double hybride et la co-immunoprécipitation ont été menées par différentes équipes, le crible étant négatif (perte d'interaction). Le domaine C-terminal de la protéine P du virus humain ou bovin était suspecté jouer un rôle important dans l'interaction avec la protéine N (Garcia Barreno et al., 1996; Mallipeddi et al., 1996 ; Slack and Easton, 1998 ; Khattar et al., 2001a, 2001b). Cependant il a été avancé que le système double hybride ne reflèterait pas la nature réelle des interactions entre les protéines N et P (Khattar et al., 2001a). En outre, la nature exacte des complexes P-N (nombre de chaque molécule ou stoechiométrie, structure) n'était pas décrite et les domaines d'interaction pas démontrés ni caractérisés.

Des études menées sur des virus proches appartenant à la famille des Paramyxoviridae (virus de Sendai, Rougeole ou Measle virus) ont conduit à l'idée que la protéine P formerait un complexe soluble avec la protéine N, noté N°P, l'empêchant de se fixer de façon non spécifique aux ARNs cellulaires (Kolakofsky et al., 2004). La protéine P serait également capable de reconnaître la nucléocapside formée de l'ARN encapsidé par la protéine N, puisque P agit comme co-facteur de L, lui permettant de « trouver » son substrat.

Pour les Paramyxoviridae, deux domaines d'interaction ont été trouvés sur P. Le premier, situé en position C-terminale de la protéine, constituerait le domaine reconnaissant le complexe N-ARN, celui situé en position N-terminale permettant la formation de complexes N°P (Kolakowsky et al., 2004). Pour le VRS, ces complexes n'ont pas été clairement identifiés, et le rôle du domaine C-terminal de la protéine P interagissant avec N n'a pas été clairement défini.

Jusqu'à présent le développement d'un vaccin de sous-unité basé sur la protéine N a été rendu impossible par la difficulté à isoler la protéine N sous forme soluble.

L'article Mitra-Kaushik et al. (2001) Virology 279:210-220 décrit la production des protéines N du virus de la peste bovine (RPV) et du virus de la peste des petits ruminants (PPRV), qui appartiennent à la sous-famille des *Paramyxovirinae,* chez *Escherichia coli.* Toutefois ces protéines N recombinantes, sous forme soluble, s'assemblent en structures analogues à des nucléocapsides en absence d'ARN viral et ne sont donc pas sous forme de ribonucléocapsides.

L'article Tan et al. (2004) J. Med. Virology 73:105-112 décrit la production, chez *Escherichia coli,* de protéines N du virus de Nipah (NiV), un membre de la sous-famille des *Paramyxovirinae,* qui s'assemblent sous forme de particules en forme de chevrons, en l'absence d'ARN viral, et ne sont donc pas sous forme de ribonucléocapsides.

L'article Choudhary et al. (2002) Virology 302:373-382 décrit un procédé de préparation d'un complexe soluble protéine N-protéine P tronquée du virus parainfluenza humain de type 3 (HPIV 3) qui appartient à la sous-famille des *Paramyxovirinae.*

Récemment les inventeurs ont développé un système de co-expression des protéines P et N en bactérie, en choisissant comme modèle de Paramyxoviridae le RSV (Castagné et al., 2004). La protéine P a été fusionnée à la glutathione-S-transferase (GST) dans un plasmide portant la résistance à l'ampicilline : la protéine N a été clonée dans un plasmide portant la résistance à la kanamycine. La co-expression de ces plasmides dans les mêmes bactéries a permis de purifier la protéine de fusion GST-P et d'entraîner avec la protéine N.

Toutefois, sans doute en raison de problèmes de solubilité subsistant, les rendements de production de protéine N sous forme soluble restent largement insuffisants pour envisager une mise en pratique de ce système à l'échelle industrielle. En outre la nature de la protéine N ainsi produite n'a pas été caractérisée.

Les inventeurs ont mis en évidence que la co-expression de mutants de délétion N-terminale de la protéine P avec la protéine N du RSV permet de purifier des grandes quantités de protéine N largement supérieure à celle obtenue avec la protéine P entière.

### Définitions

La famille des « *Paramyxoviridae* » regroupe les sous-familles des *Paramyxovirinae* et des *Pneumovirinae.* Les *Paramyxovirinae* incluent les Respirovirus, dont le virus prototype est le virus de Sendai, et les Rubulavirus (notamment le virus des oreillons), et les Morbilivirus tels que le virus de la rougeole. Chacun des genres Respirovirus et Rubulavirus regroupe des souches du virus parainfluenza. La sous-famille des *Pneumovirinae* regroupe deux genres, les Pneumovirus et les Metapneumovirus, ce dernier incluant le Metapneumovirus humain. Le virus respiratoire syncytial (VRS) humain constitue le virus prototype du genre Pneumovirus appartenant à la sous-famille des *Pneumovirinae.* Les Pneumovirus incluent également les souches bovine et murine du VRS.

Sans autre précision, par « virus respiratoire syncytial » on entend d'une manière générale le VRS, quelle que soit la forme (humaine, bovine ...), le sous-groupe (par exemple les sous-groupes A, B et S identifiés chez le VRS humain) ou la souche considérée.

L'expression « protéine P » désigne la Phosphoprotéine ou protéine P faisant partie du complexe Polymérase d'un virus de la famille des *Paramyxoviridae.* La protéine P est un co-facteur de la polymérase (réplicase/transcriptase) virale et peut être phosphorylée. Les séquences de protéines P de *Paramyxoviridae* sont connues de l'homme du métier. Par exemple la protéine P du VRS humain souche Long présente une séquence de 241 acides aminés qui a été déposée dans la base de données Swissprot sous le numéro d'accès P12579. Cette séquence est montrée dans la séquence SEQ ID No.1. La protéine P du VRS bovin comporte également 241 acides aminés (SEQ ID No.23). Une protéine P du virus de Sendai (souche Harris), du virus de la rougeole (Souche Edmonston B), du virus de oreillons (souche SBL-1), et du Metapneumovirus humain (souche 00-1) sont également décrites dans la base de données Swissprot sous les numéros d'accès P04859 (SEQ ID No.2), CAA91364 (SEQ ID No.3), P19717 (SEQ ID No.4), et Q91KZ5 (SEQ ID No.5), respectivement. L'expression « protéine P » peut désigner une protéine P entière, une protéine P tronquée ou un fragment de la protéine P.

La protéine P des *Paramyxoviridae* forme des homo-oligomères, en particulier des homo-tétramères par exemple chez le virus de Sendai ou le VRS. Pour le VRS, un domaine de la protéine P capable de s'oligomériser (oligomérisation P-P) a été cartographié au niveau des acides aminés 120 à 150 de cette protéine (Castagné et al., 2004). Ainsi, par exemple, le fragment constitué par les acides aminés 161 à 241 de la protéine P du VRS ne forme pas d'oligomères. Le domaine d'oligomérisation de la protéine P du virus de Sendai a été décrit par Tarbouriech et al. (2000) comme étant constitué par les résidus 320 à 446 de la protéine P. D'autre part, la région d'oligomérisation P à été identifiée au niveau des acides aminés 304-376 pour la protéine P du virus de la rougeole (Johansson et al., 2003).

L'expression « protéine P tronquée » désigne une protéine P dans laquelle une ou plusieurs séquences d'acides aminés contigus ont été supprimées. Il peut s'agir de la troncature d'une séquence C-terminale, d'une séquence N-terminale, d'une séquence « interne » par rapport à la séquence primaire de la protéine P, ou d'une combinaison de ces troncatures.

Les protéines P tronquées selon l'invention ne contiennent pas le domaine d'oligomérisation P et sont capable d'interagir avec la protéine N. Comme le domaine d'interaction de la protéine P des *Paramyxoviridae* avec la protéine N a été cartographié au niveau de l'extrémité C-terminale, des exemples de protéine P tronquée incluent préférentiellement un fragment C-terminal de la protéine P, ou une protéine P « chimère » constituée par la fusion d'un fragment C-terminal de la protéine P (capable d'interagir avec la protéine N) avec au moins une autre séquence d'acides aminés contigus de la protéine P. Ledit fragment C-terminal et ladite autre séquence de la protéine P ne sont pas eux-mêmes naturellement contigus, et ne présentent pas de recouvrement de séquence entre eux. Par exemple, une protéine P tronquée du VRS peut avoir la séquence constituée par les acides aminés 1 à 121 et 161 à 241 de la protéine P native.Un « fragment » d'un polypeptide de référence désigne n'importe quelle séquence d'acides aminés contigus trouvés dans la séquence du polypeptide de référence.

Par « fragment de la protéine P » ou « PΔ » on entend un polypeptide dont la séquence comprend un enchaînement d'acides aminés de la protéine P, un ou plusieurs acides aminés consécutifs de la protéine P ayant été supprimés à partir de l'extrémité N-terminale et/ou C-terminale.

Par « fragment C-terminal de la protéine P » ou « PΔN » on entend une protéine P dans laquelle un ou plusieurs acides aminés consécutifs ont été supprimés à partir de l'extrémité N-terminale. De préférence, un fragment C-terminal de la protéine P désigne un enchaînement d'acides aminés positionnés dans la moitié C-terminale de la séquence primaire de la protéine P (lorsque le nombre d'acides aminés de la séquence est impaire, on peut attribuer arbitrairement un acides aminé supplémentaire à la moitié C-terminale de la protéine par rapport à la moitié N-terminale). Par exemple, pour la protéine P du VRS qui comprend 241 acides aminés, PΔ161N désigne un fragment C-terminal constitué par les acides aminés 161 à 241 de la protéine P. Par exemple également, pour la protéine P du virus de la rougeole (souche Edmonston B) qui comprend 507 acides aminés, PΔ386N désigne un fragment C-terminal constitué par les acides aminés 386 à 507 de la protéine P.

Par « fragment N-terminal de la protéine P » ou « PΔC » on entend une protéine P dans laquelle un ou plusieurs acides aminés consécutifs ont été supprimés à partir de l'extrémité C-terminale.

Par « fragment interne de la protéine P » ou « PΔNC » on entend une protéine P dans laquelle un ou plusieurs acides aminés consécutifs ont été supprimés à partir de l'extrémité N-terminale et un ou plusieurs acides aminés consécutifs ont été supprimés à partir de l'extrémité C-terminale.

Par « protéine N » on désigne la protéine de nucléocapside des *Paramyxoviridae* qui forme des structures hélicoïdales pour entourer le génome viral. La protéine N du VRS humain souche Long présente une séquence de 391 acides aminés qui est décrite dans la séquence SEQ ID No.6. La protéine N du VRS bovin comporte également 391 acides aminés (voir SEQ ID No. 24). Une protéine N du virus de Sendai (souche Hamamatsu), du virus de la rougeole (Souche Edmonston B), du virus de oreillons (souche SBL-1), et du Metapneumovirus humain (souche 00-1) sont également décrites dans la base de données Swissprot sous les numéros d'accès Q9DUE3 (SEQ ID No.7), Q89933 (SEQ ID No.8), P21277 (SEQ ID No.9), et Q91 F57 (SEQ ID No.10), respectivement.

Les séquences des protéines P et N décrites ci-dessus ont un caractère illustratif, ces séquences étant susceptibles de présenter des variations en fonction de la souche particulière considérée pour un virus donné. Ainsi, les positions d'acides aminés mentionnées dans la suite de la demande sont indiquées par rapport à ces séquences de référence. L'homme du métier est tout à fait à même d'identifier les domaines correspondants dans des souches de virus autres que celles exemplifiées.

Les séquences codantes de ces protéines N et P de virus de la famille des *Paramyxoviridae* sont également connues de l'homme du métier.

Par « protéine étiquette », aussi appelée « protéine tag », on désigne une protéine qui est utilisée en fusion avec une protéine d'intérêt pour en faciliter la purification. Des protéines étiquettes sont connues de l'homme du métier. Des exemples de protéines étiquette incluent la gluthation-S-transférase (GST) ou les tag histidine qui sont des séquences comprenant généralement un enchaînement de 4 à 10 résidus histidine.

Dans le contexte de l'invention, le terme « homologue » a trait à la relation existant entre des protéines qui possèdent une même origine évolutive, par exemples des protéines homologues appartenant à différentes espèces, ou dans le cas de virus, de souches virales. De telles protéines (et leurs gènes codant), présentent des homologies de séquence, reflétées par leur similarité de séquences, que ce soit en terme de pourcentage de similitude ou en terme de présence de résidus ou motifs spécifiques au niveau de postions conservées.

L'expression « similarité de séquences » désigne le degré d'identité entre des séquences d'acides nucléiques ou d'acides aminés de protéines qui peuvent ou non partager une même origine évolutive. De manière usuelle, on utilise indistinctement les termes homologie ou similarité. Deux séquences d'acides aminés sont « essentiellement homologues » lorsque leurs acides aminés sont identiques à au moins 80%, ou similaires (c'est-à-dire fonctionnellement identiques) à au moins 90 %. Des séquences similaires ou homologues peuvent être identifiées par alignement, en utilisant par exemple les programmes BLAST ou FASTA.

La solubilité des protéines ou complexes selon l'invention est définie par rapport à un milieu aqueux tamponné tel que le PBS 1X ; un tampon Tris 10 mM pH 7,4 - 8,0, NaCl 150 mM ; du TBE 0,2 X, ou encore par exemple un tampon de lyse de bactéries comprenant 50 mM Tris-HCI pH 7,8, 60 mM NaCl, 1 mM EDTA, 2 mM DTT, 0,2% Triton X-100, 10 mM MgSO4, 1 mM CaCl2, et 1 mg/ml lysozyme.

### Procédé de préparation de complexe soluble protéine N - protéine P tronquée

Les inventeurs ont précédemment montré (Castagné et al., 2004) que la co-expression de plasmides codant respectivement une fusion de la protéine P avec la GST et la protéine N d'un *Paramyxoviridae,* permettait de recueillir la protéine de fusion GST-P en entraînant la protéine N. Les rendements de production de complexes N-P sont toutefois faibles au point de ne pas être conciliables avec une production à l'échelle industrielle de ces complexes.

Les inventeurs ont caractérisé des mutants de délétion de la protéine P en déterminant leur capacité à interagir avec la protéine N. Ils ont ainsi mis en évidence que certains de ces mutants sont à la fois capables d'interagir avec la protéine N sous forme de complexe ARN-N ou ribonucléocapside (RNP), mais aussi de permettre la préparation de complexes N-P avec des rendements de préparation très nettement supérieurs à ceux obtenus avec la protéine P entière. Ces mutants particuliers correspondent à des fragments de la protéine P comprenant la partie C-terminale de la molécule et dans lesquels le domaine d'oligomérisation P est absent.

La coexpression de ces mutants de la protéine P avec la protéine N permet donc de préparer de la protéine N sous forme de RNP soluble, notamment sous forme de complexes protéine N-protéine P tronquée, en particulier fragment C-terminal de P, en grandes quantités.

Est décrit ici un procédé de préparation d'un complexe soluble protéine N -protéine P tronquée d'un virus de la famille des *Paramyxoviridae,* ledit procédé comprenant les étapes consistant à :
a) coexprimer une protéine N d'un virus de la famille des *Paramyxoviridae* avec une protéine P tronquée du même virus de la famille des *Paramyxoviridae,* ladite protéine P tronquée ne contenant pas le domaine d'oligomérisation P et étant capable d'interagir avec la protéine N ;
b) recueillir les complexes solubles protéine N -protéine P tronquée ainsi formés.

La présente invention concerne en particulier un procédé de préparation d'un complexe soluble protéine N -protéine P tronquée d'un virus de la famille des *Paramyxoviridae,* ledit procédé comprenant les étapes consistant à :
a) coexprimer une protéine N d'un virus de la famille des *Paramyxoviridae* avec une protéine P tronquée du même virus de la famille des *Paramyxoviridae,* ladite protéine P tronquée ne contenant pas le domaine d'oligomérisation P et étant capable d'interagir avec la protéine N ;
b) recueillir les complexes solubles protéine N -protéine P tronquée ainsi formés;
dans lequel le virus de la famille des *Paramyxoviridae* est un virus de la sous-famille des *Pneumovirinae.*

La protéine P tronquée comprend préférentiellement un fragment C-terminal de la protéine P. Le domaine d'interaction de la protéine P des *Paramyxoviridae* avec la protéine N, éventuellement sous forme de complexe N-ARN, est en effet localisé du côté C-terminal de la protéine P.

La protéine P tronquée peut être une protéine P « chimère » constituée par la fusion d'un fragment C-terminal de la protéine P avec au moins une autre séquence d'acides aminés contigus de la protéine P, comme défini plus haut.

De préférence la protéine P tronquée est un fragment C-terminal de la protéine P.

Est ainsi décrit un procédé de préparation d'un complexe soluble protéine N - fragment C-terminal de la protéine P ("complexe N-PΔN") d'un virus de la famille des *Paramyxoviridae,* ledit procédé comprenant les étapes consistant à :
a) coexprimer une protéine N d'un virus de la famille des *Paramyxoviridae* avec un fragment C-terminal de la protéine P du même virus de la famille des *Paramyxoviridae,* ledit fragment C-terminal de la protéine P ne contenant pas le domaine d'oligomérisation P et étant capable d'interagir avec la protéine N ;
b) recueillir les complexes solubles N-PΔN ainsi formés.

Ledit virus de la famille des *Paramyxoviridae* peut être un *Paramyxovirinae* ou un *Pneumovirinae.* En particulier, le virus peut être sélectionné dans le groupe constitué du virus des oreillons, du virus de la rougeole, du Metapneumovirus humain et du virus parainfluenza. De préférence, le virus est un *Pneumovirus* tel que le virus respiratoire syncytial (VRS), humain ou bovin.

L'homme du métier connaît ou est à même de déterminer des protéines P tronquées, ou plus spécifiquement des fragments C-terminaux de la protéine P, qui sont capables d'interagir avec la protéine N.

Par exemple, dans le cas du VRS, les inventeurs ont utilisé la stratégie de co-expression des protéines N et P chez *E. coli* précédemment décrite (Castagné et al., 2004) pour cartographier le domaine d'interaction entre P et N. Pour cela, la protéine N a été coexprimée avec des mutants de délétion de P fusionnés avec la GST. Les inventeurs ont ainsi mis en évidence que le domaine d'interaction de P avec N est localisé au niveau de l'extrémité C-terminale de la protéine P (Figure 1). Les inventeurs ont plus particulièrement montré que des fragments C-terminaux de P, jusqu'à un oligopeptide constitué par les 9 acides aminés C-terminaux de la protéine P du VRS (acides aminés 233 à 241), sont capables d'interagir avec la protéine N.

Il a d'autre part été décrit, par exemple, que le domaine d'interaction de la protéine P du virus de Sendai, avec la protéine N sous forme de complexe ARN-N ou RNP, appelé « X-domain » ou XD, est défini par les acides aminés 473 à 568 (Kolakofsky et al. 2004).

Pour les autres *Paramyxoviridae,* le cas échéant, l'homme du métier est à même d'identifier sur la protéine P le domaine d'interaction avec la protéine N sous forme de nucléocapside en utilisant la stratégie décrite par les inventeurs.

Les inventeurs ont également mis en évidence que certains fragments C-terminaux de la protéine P du VRS, notamment le fragment PΔ161N (acides aminés 161 à 241), permettaient de préparer de grandes quantités de protéine N, comparativement à la protéine P entière qui, en pratique, ne permet pas d'obtenir des rendements suffisants à une échelle industrielle. Les mutants de délétion plus petits, jusqu'à PΔ233N (acides aminés 233 à 241) qui ne contient plus que 9 acides aminés, permettent d'obtenir des rendements comparables à ceux de PΔ161N.

Ces fragments plus petits que PΔ161N correspondent à des fragments de la protéine du VRS capables d'interagir avec la protéine N et qui ne sont plus capables de s'oligomériser, donc ne contiennent plus le domaine d'oligomérisation P. Le domaine minimum d'oligomérisation P du VRS serait en effet défini par environ les acides aminés 120 à 150 de la protéine P.

Cette même stratégie a permis aux inventeurs de montrer qu'un fragment C-terminal de la protéine P du virus de la rougeole, constitué par les résidus d'acides aminés 386-507 (PΔ386N), interagissait avec la protéine N de ce virus et permettait sa purification. En revanche une délétion de la partie N-terminale de la protéine P, jusqu'à hauteur du résidu 456 (inclus ; fragment PΔ457N), ne permet pas de purifier la protéine N. La structure de la région C-terminale de la protéine P interagissant avec la ribonucléocapside a été déterminée par Johansson et al. (2003). La région d'oligomérisation P a été déterminée par délétions et prédiction comme étant définie par les acides aminés 304-376.

L'utilisation de fragments C-terminaux de la protéine P qui contiennent le domaine d'interaction avec la protéine N sous forme de RNP mais dans lesquels le domaine d'oligomérisation P a été délété permet donc à la fois l'interaction des fragments de P avec N et la formation de complexes solubles N-PΔN, et la production de ces complexes avec un rendement élevé. Sans vouloir se lier à un mécanisme particulier, il est supposé que l'absence du domaine d'oligomérisation P évite des problèmes d'insolubilité des complexes N-ΔPN liés à des interactions entre protéines P de ces complexes.

Ainsi, selon un mode de réalisation, le procédé de préparation de complexe N-PΔN implique l'expression d'un fragment C-terminal de la protéine P du VRS qui comprend les 9 derniers acides aminés C-terminaux de la protéine P du VRS et qui est dépourvu d'au moins les 119, de préférence, les 149, de préférence encore les 160 acides aminés N-terminaux de la protéine P du VRS.

Plus spécifiquement, dans le procédé selon l'invention on peut coexprimer avec la protéine N du VRS :
a) un fragment C-terminal de la protéine P du VRS qui comprend la séquence d'acides aminés 233 à 241 de la protéine P du VRS humain souche LONG telle que montrée dans SEQ ID No.1, et qui s'étend en direction N-terminale jusqu'à un résidu d'acide aminé compris entre les positions 233 et 120, de préférence 150, de préférence encore 161, de la séquence de la protéine P du VRS telle que montrée dans SEQ ID No.1, ou
b) un fragment C-terminal, homologue du fragment défini en a), d'une protéine P issue d'une autre souche du VRS humain ou d'une souche du VRS bovin.

Le fragment C-terminal de la protéine P du VRS peut par exemple être sélectionné dans le groupe constitué de PΔ120N (acides aminés 120 à 241 de P), PΔ150N (acides aminés 150 à 241 de P), PΔ161N (acides aminés 161 à 241 de P), PΔ180N (acides aminés 180 à 241 de P), PΔ200N (acides aminés 200 à 241 de P), PΔ220N (acides aminés 220 à 241 de P), PΔ230N (acides aminés 230 à 241 de P) et PΔ233N (acides aminés 233 à 241 de P).

L'invention concerne également un procédé dans lequel on coexprime avec la protéine N du VRS une protéine P tronquée comprenant un fragment C-terminal de la protéine P du VRS tel que décrit ci-dessus, qui comprend les 9 derniers acides aminés C-terminaux de la protéine P du VRS et qui ne comprend pas au moins les 119, de préférence, les 149, de préférence encore les 160 acides aminés N-terminaux de la protéine P du VRS.

Par exemple la protéine P tronquée comprenant un fragment C-terminal de la protéine P peut être constituée par la fusion des 122 derniers acides aminés N-terminaux avec les 80 derniers acides aminés C-terminaux de la protéine P du VRS ; elle peut par exemple être constituée par l'enchaînement des acides aminés 1 à 121 et 161 à 241 de la protéine P du VRS humain souche LONG telle que montrée dans SEQ ID No.1.

Dans un exemple exdu de l'invention, le *Paramyxoviridae* est le virus de la rougeole et le procédé de préparation de complexe N-PΔN implique l'expression d'un fragment C-terminal de la protéine P du virus de la rougeole comprenant au plus, ou constitué de, les 122 acides aminés C-terminaux de la protéine P. En particulier, il peut s'agir d'un fragment C-terminal constitué par les acides 386 à 507 de la protéine P (PΔ386N) du virus de la rougeole souche Edmonston B, telle que montrée dans SEQ ID No.3, ou d'un fragment C-terminal, homologue à celui défini pour protéine P de la souche Edmonston, d'une protéine P issue d'une autre souche de virus de la rougeole.

Pour mettre en oeuvre le procédé selon l'invention n'importe quelle technique conventionnelle de biologie moléculaire, microbiologie ou d'ADN recombinant peuvent être employées. De telles techniques sont à la portée de l'homme du métier et ont été décrites, notamment dans Sambrook, Fritsch & Maniatis, Molecular Cloning: A Laboratory Manual, Second Edition (1989) Cold Spring Harbor Laboratory Press, Cold Spring Harbor, New York ("Sambrook et al., 1989") ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et al. (eds.), Current Protocols in Molecular Biology, John Wiley & Sons, Inc. (1994).

« Exprimer » ou « expression » signifient permettre ou faire en sorte que l'information contenue dans un gène ou une séquence d'ADN devienne manifeste, par exemple en produisant une protéine par activation des fonctions cellulaires impliquées dans la transcription et la traduction de la séquence génétique ou ADN correspondante. On parle de « coexpression » lorsque l'information contenue dans deux gènes ou séquences d'ADN est exprimée dans un même hôte cellulaire.

Une « séquence codante » désigne une séquence nucléotidique qui, lorsqu'elle est exprimée, résulte en la production d'un ARN, d'un polypeptide, d'une protéine, etc. Une séquence codante d'une protéine contient généralement un codon d'initiation (ATG) et un codon stop.

Une séquence codante est « sous le contrôle de » ou « associée de manière fonctionnelle avec » des séquences de contrôle transcriptionnelles et traductionelles lorsque une ARN polymérase transcrit la séquence codante en ARN, en particulier en ARNm, qui peut ensuite être épissé s'il contient des introns, et traduit en la protéine codée par la séquence codante.

Les expressions « vecteur », « vecteur de clonage » et « vecteur d'expression » désignent le véhicule par lequel une séquence d'ADN ou d'ARN (par exemple un gène hétérologue) peut être introduite dans une cellule hôte de manière à transformer la cellule hôte et à promouvoir l'expression de la séquence introduite. Des exemples de vecteurs incluent les plasmides, les phages, les virus. Les vecteurs les plus communs sont les plasmides qui sont des unités de réplication autonomes, en général d'origine bactérienne, et qui peuvent être sous forme d'ADN double brin. Les plasmides peuvent intégrer facilement une séquence d'ADN exogène qui peut alors être facilement introduite dans un hôte approprié. Un vecteur plasmide contient généralement une séquence d'ADN codante, une séquence d'ADN promotrice et présente un ou plusieurs sites de restriction permettant d'introduire un ADN exogène. Des exemples non limitatifs de plasmides incluent les plasmides pKK (Clonetech), pUC et pET (Novagen, Inc., Madison, WI), pRSET ou pREP (Invitrogen, San Diego, CA), pMAL (New England Biolabs, Beverly, MA), ou pGEX-4T-3 (Pharmacia).

Par « cellule hôte » ont entend n'importe quelle cellule ou organisme qui est sélectionné, modifié, cultivé ou manipulé, pour la production d'une substance par la cellule, par exemple l'expression par la cellule d'un gène, d'une séquence ADN ou ARN, d'une protéine ou d'une enzyme.

Un « système d'expression » désigne une cellule hôte et un vecteur compatible utilisés dans des conditions appropriées pour produire une protéine codée par un ADN exogène porté par le vecteur et introduit dans la cellule hôte. Des systèmes d'expressions usuels incluent des cellules hôte *E. coli* et des vecteurs plasmides, des cellules d'insecte et des vecteurs Baculovirus ou encore des cellules de mammifère et des vecteurs.

Le système d'expression selon le procédé de l'invention est avantageusement un système d'expression bactérien, en particulier dans *E*. *coli,* avec par exemple comme vecteur pGEX-4T-3. Les systèmes bactériens sont en effet lés systèmes d'expression qui permettent généralement d'obtenir les rendements de production les plus élevés.

Avantageusement la protéine P tronquée, et en particulier le fragment C-terminal de la protéine P, est exprimé sous forme de fusion avec une protéine facilitant la purification des complexes N-protéine P tronquée, notamment une protéine utilisable en chromatographie d'affinité. Il peut s'agir d'une protéine étiquette telle que la gluthation-S-transférase (GST), auquel cas la protéine de fusion protéine P tronquée-GST peut être isolée par chromatographie sur un support solide couplé au glutathion. D'autres étiquettes «ou « Tag » peuvent être utilisés comme le poly-histidine ou « His-Tag ».

On obtient ainsi des complexes N-protéine P tronquée-protéine étiquette (GST ou autre protéine étiquette en fusion avec la protéine P tronquée, en particulier le fragment PΔN), dans lesquels la protéine étiquette peut être éliminée par clivage enzymatique. Par exemple, la GST peut être éliminée par clivage à la thrombine, ou par tout autre enzyme appropriée lorsque la fusion comprend une protéine autre que la GST.

Des exemples spécifiques de construction de vecteurs permettant de mettre en oeuvre le procédé selon l'invention sont décrits dans les exemples ci-dessous.

### Complexes solubles N-protéine P tronquée

Le procédé de préparation d'un complexe soluble protéine N - protéine P tronquée, en particulier fragment C-terminal de la protéine P, tel que décrit ci-dessus permet aisément d'obtenir des complexes N-protéine P tronquée sous forme isolée ou purifiée.

Est ainsi décrit un complexe soluble protéine N - protéine P tronquée d'un virus de la famille des *Paramyxoviridae,* susceptible d'être obtenu par un procédé de préparation selon l'invention.

La présente invention concerne ainsi un complexe isolé soluble protéine N - protéine P tronquée d'un virus de la famille des *Paramyxoviridae,* ladite protéine N étant sous forme de ribonucléocapside et ledit virus de la famille des *Paramyxoviridae* étant un virus de la sous-famille des *Pneumovirinae*, ladite protéine P tronquée ne contenant pas le domaine d'oligomérisation P et étant capable d'interagir avec la protéine N.

De préférence, la protéine P tronquée comprend ou est un fragment C-terminal de la protéine P.

Est plus particulièrement décrit un complexe soluble protéine N - fragment C-terminal de la protéine P ("complexe N-PΔN") d'un virus de la famille des *Paramyxoviridae,* susceptible d'être obtenu par un procédé de préparation décrit ici.

Ledit virus de la famille des *Paramyxoviridae* peut être un *Paramyxovirinae* ou un *Pneumovirinae.* En particulier, le virus peut être sélectionné dans le groupe constitué du virus des oreillons, du virus de la rougeole, du Metapneumovirus humain (HMPV) et du virus parainfluenza. De préférence, le virus est un *Pneumovirus* tel que le virus respiratoire syncytial (VRS), par exemple humain ou bovin.

Selon un mode de réalisation, le virus *Paramyxoviridae* est le virus respiratoire syncytial (VRS) et ledit fragment C-terminal de la protéine P comprend les 9 derniers acides aminés C-terminaux de la protéine P du VRS et est dépourvu d'au moins les 119, de préférence, les 149, de préférence encore les 160 acides aminés N-terminaux de la protéine P du VRS.

Plus spécifiquement ledit fragment C-terminal de la protéine P peut comprendre
a) la séquence d'acides aminés 233 à 241 de la protéine P du VRS humain souche LONG telle que montrée dans SEQ ID No.1, et s'étendre en direction N-terminale jusqu'à un résidu d'acide aminé compris entre les positions 233 et 120, de préférence 150, de préférence encore 161, de la séquence de la protéine P du VRS telle que montrée dans SEQ ID No.1, ou
b) un fragment C-terminal, homologue du fragment défini en a), d'une protéine P issue d'une autre souche du VRS humain ou d'une souche du VRS bovin.

Il peut s'agir en particulier d'un fragment C-terminal de la protéine P sélectionné dans le groupe constitué de PΔ120N, PΔ150N, PΔ161N, PΔ180N, PΔ200N, PΔ220N, PΔ230N et PΔ233N.

L'invention concerne également un complexe soluble contenant une protéine P tronquée du VRS comprenant un fragment C-terminal de la protéine P du VRS tel que décrit ci-dessus, qui comprend les 9 derniers acides aminés C-terminaux de la protéine P du VRS et qui est dépourvu d'au moins les 119, de préférence, les 149, de préférence encore les 160 acides aminés N-terminaux de la protéine P du VRS.

Par exemple la protéine P tronquée du VRS comprenant un fragment C-terminal de la protéine P peut être constituée par la fusion des 122 derniers acides aminés N-terminaux avec les 80 derniers acides aminés C-terminaux de la protéine P du VRS ; elle peut par exemple être constituée par l'enchaînement des acides aminés 1 à 121 et 161 à 241 de la protéine P du VRS humain souche LONG telle que montrée dans SEQ ID No.1.

Dans un exemple exclu de l'invention, le virus *Paramyxoviridae* est le virus de la rougeole et ledit fragment de la protéine P est un fragment C-terminal de la protéine P qui comprend au plus, ou est constitué de, les 122 acides aminés C-terminaux de la protéine P. Plus spécifiquement, ledit fragment C-terminal de la protéine P du virus de la rougeole peut être constitué par les addes 386 à 514 de la protéine P (PΔ386N) du virus de la rougeole souche Edmonston B, telle que montrée dans SEQ ID No.3, ou être un fragment C-terminal, homologue à celui défini pour protéine P de la souche Edmonston, d'une protéine P issue d'une autre souche de virus de la rougeole.

Dans le complexe N-PΔN, la protéine PΔN peut éventuellement être présente sous forme de fusion avec une protéine étiquette, par exemple la GST, un tag histidine, ou tout autre protéine appropriée facilitant la purification des complexes N-PΔN.

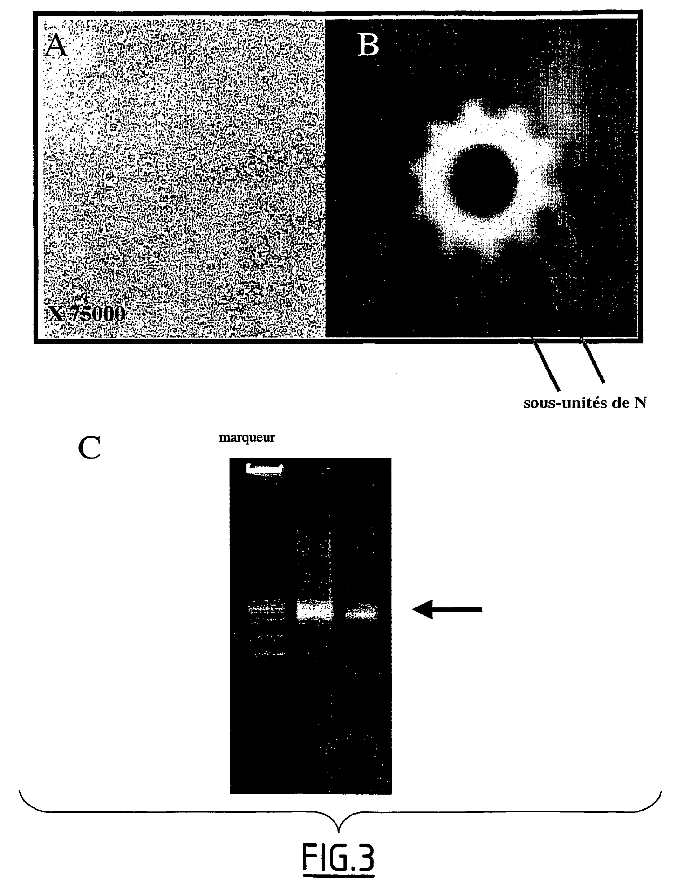

L'analyse au microscope électronique des complexes produits pour le VRS a révélé qu'ils étaient composés d'anneaux contenant 10 molécules de protéine N, ces anneaux solubles contenant également un petit ARN d'environ 70 bases, visible par électrophorèse en gel d'agarose (Figure 3). Dans ces complexes, on trouve une quantité similaire de protéines N et de protéines PΔN-GST. L'ARN ne peut être dissocié de l'anneau de protéine N sans dénaturation de cette protéine.

Ainsi, l'invention a trait également à un complexe isolé soluble protéine N - fragment C-terminal de la protéine P du virus respiratoire syncytial (VRS), comprenant 10 molécules de protéine N, chacune, ou la majorité d'entre elles, étant associées à un fragment C-terminal de la protéine P, dans lequel ledit fragment C-terminal de P comprend les 9 derniers acides aminés C-terminaux de la protéine P du VRS et est dépourvu d'au moins les 160 acides aminés N-terminaux de la protéine P du VRS, comme défini ci-dessus. Ce complexe N-PΔN du VRS comprend en outre un ARN d'environ 70 bases.

### Procédé de préparation de protéine N solubles

A partir de ces complexes N -protéine P tronquée, ou plus spécifiquement N-PΔN, la protéine N soluble peut être aisément isolée sous forme d'anneaux, avec leur ARN, par exemple par chromatographie d'exclusion de taille (gel filtration). Cette séparation peut être mise en oeuvre, le cas échéant, après séparation, par clivage enzymatique, de la protéine P tronquée, et de la protéine étiquette à laquelle la protéine P tronquée est éventuellement fusionné.

Ici est en outre décrit un procédé de préparation de protéines N solubles d'un virus de la famille des *Paramyxoviridae,* ledit procédé comprenant les étapes consistant à :
a) préparer un complexe soluble protéine N - protéine P tronquée par un procédé tel que défini plus haut; et
b) séparer les protéines N solubles des complexes solubles N-protéine P tronquée.

La présente invention concerne en particulier un procédé de préparation de protéines N solubles d'un virus de la famille des *Paramyxoviridae,* ledit procédé comprenant les étapes consistant à :
a) préparer un complexe soluble protéine N -protéine P tronquée par un procédé de préparation selon l'invention ; et
b) séparer les protéines N solubles des complexes solubles protéine N - protéine P tronquée ;
dans lequel le virus de la famille des *Paramyxoviridae* est un virus de la sous-famille des *Pneumovirinae.*

Préférentiellement, la protéine P tronquée comprend ou est un fragment C-terminal de la protéine P.

Ici est plus particulièrement décrit un procédé de préparation de protéines N solubles d'un virus de la famille des *Paramyxoviridae,* ledit procédé comprenant les étapes consistant à :
a) préparer un complexe soluble protéine N - fragment C-terminal de la protéine P ("complexe N-PΔN") par un procédé tel que défini plus haut; et
b) séparer les protéines N solubles des complexes solubles N-PΔN.

Ledit virus de la famille des *Paramyxoviridae* peut être un *Paramyxovirinae* ou un *Pneumovirinae.* En particulier, le virus peut être sélectionné dans le groupe constitué du virus des oreillons, du virus de la rougeole, du Métapneumovirus humain et du virus parainfluenza. De préférence, le virus est un *Pneumovirus* tel que le virus respiratoire syncytial (VRS), par exemple humain ou bovin.

Les protéines N solubles d'un virus de la famille des *Paramyxoviridae,* susceptibles d'être obtenues par le procédé ci-dessus sont également décrites ici.

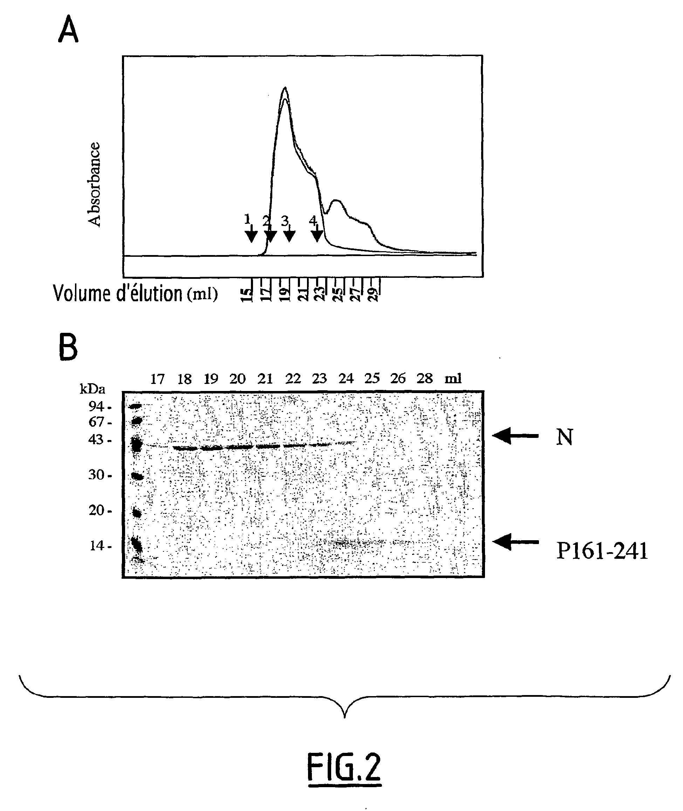

Dans le cas du VRS, la protéine N a une masse apparente de 450 kDa alors que le plus grand des fragments C-terminaux de la protéine P utilisable (PΔ161N) a une masse de 15 kDa. Les anneaux de protéine N peuvent donc être séparés des fragments C-terminaux de protéine P par exemple par chromatographie sur colonne Sephadex comme décrit dans l'exemple 3 ci-dessous (et Figure 2).

Selon un mode de réalisation, l'invention propose donc des protéines N du VRS solubles, lesdites protéines N étant associées en anneaux ayant un diamètre d'environ 7 nm et contenant 10 sous-unités. Toutefois on ne peut exclure que certains anneaux soient partiels et contiennent moins de 10 sous-unités. Les anneaux contiennent en outre un ARN d'environ 70 bases.

La présente invention concerne en particulier des ribonucléocapsides solubles comprenant des protéines N du VRS associées sous forme d'anneaux ayant un diamètre d'environ 7 nm et convenant 10 sous-unités et contenant en outre un ARN d'environ 70 bases.

### Compositions vaccinales

La protéine N du VRS, et plus généralement des *Paramyxoviridae,* est un antigène intéressant pour la vaccination, mais, jusqu'à présent, personne n'avait réussi à la purifier sous forme soluble. Le procédé selon l'invention permet d'obtenir facilement et en grandes quantités des protéines N structurées en anneaux, très pures et très homogènes.

Afin d'évaluer les propriétés immunogènes de la protéine N en anneaux, les inventeurs ont immunisé des souris avec un complexe N-PΔN de *Paramyxoviridae* selon l'invention. Pour une utilisation vaccinale, les protéines N et PΔN peuvent éventuellement être séparées mais cette opération n'est pas nécessaire, la présence de PΔN n'ayant pas d'effet négatif.

Les inventeurs ont plus spécifiquement immunisé des souris avec le complexe N-PΔ161N du VRS et utilisé le polypeptide PΔ161N du VRS comme témoin.

Compte tenu de la spécificité de ce virus pour les voies respiratoires, deux voies d'immunisation ont été comparées : la voie sous-cutanée, qui est une voie classique de vaccination parentérale, et la voie nasale, qui permet d'induire une immunité locale au niveau de la muqueuse respiratoire et des tissus lymphoïdes associés.

Comme il est difficile d'obtenir une réponse immunitaire contre une protéine recombinante soluble en l'absence d'adjuvant de vaccination, les inventeurs ont également utilisé la lymphotoxine détoxifiée de *E. Coli,* LT(R192G) (donnée par Dr. J.D. Clements, USA) dont les propriétés adjuvantes par voie mucosale ont été bien décrites (McNeal et al. 2002, Freytag et Clements 2005).

Les paramètres de la réponse immunitaire qui ont été suivis sont (i) la production d'anticorps sériques et muqueux (par lavages broncho-alvéolaires) et (ii) la réponse cellulaire via la production d'IFN-γ par des lymphocytes T mémoires isolés de la rate ou du poumon.

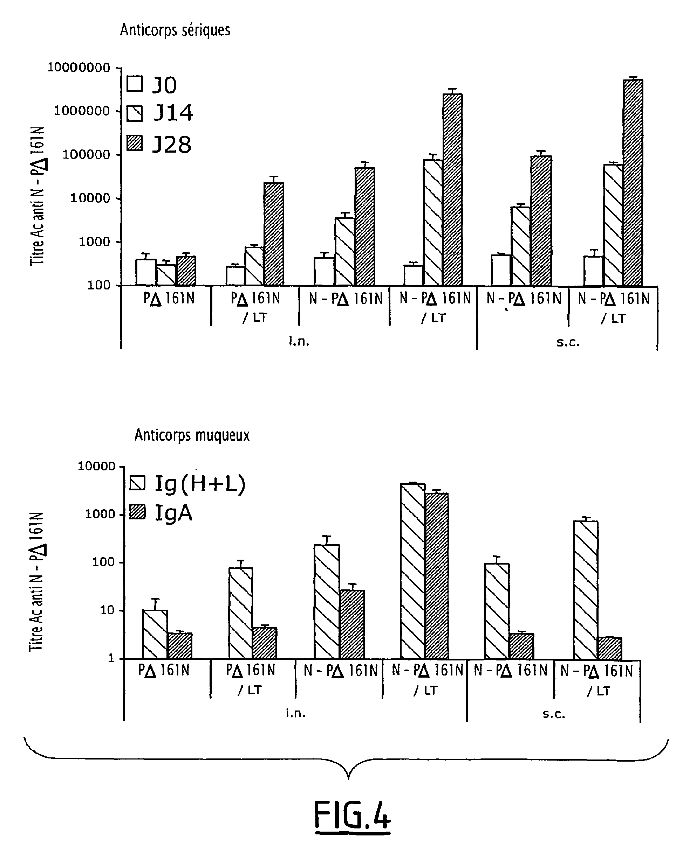

En présence de l'adjuvant LT(R192G), la protéine N (complexe N-PΔ161N) induit une forte production d'anticorps sériques quelle que soit la voie d'administration (nasale ou sous-cutanée) (Figure 4A). Cette réponse est détectable après la première immunisation (J14) et est amplifiée après le rappel (J28). L'adjuvant permet aussi d'induire après rappel une production d'anticorps contre PΔ161N.

De façon tout à fait remarquable, l'administration du complexe N-PΔ161N sans adjuvant par voie nasale et sous-cutanée génère une forte production d'anticorps sériques dès la première immunisation. Dans les mêmes conditions PΔ161N n'induit pas de réponse. Un profil de réponse comparable est observé pour la production des anticorps muqueux, Ig totales (Figure 4B).

Par contre, seule la protéine N (complexe N-PΔ161N) administrée par voie nasale en présence de LT(R192G) permet la production d'IgA au niveau de la muqueuse respiratoire (Figure 4B).

La réponse cellulaire a été mesurée par la capacité de leucocytes de la rate ou du poumon à produire de l'IFN-γ *in vitro* en présence de N-PΔ161N ou PΔ161 seul. L'IFN-γ est une cytokine produite par les lymphocytes T CD4 et CD8. Des lymphocytes T CD4 et CD8 mémoires peuvent être réactivés *in vitro* en présence de l'antigène dont ils sont spécifiques. L'IFN-γ est produit par les lymphocytes Th1 et les lymphocytes T cytotoxiques, qui sont les effecteurs majeurs des défenses anti-virales.

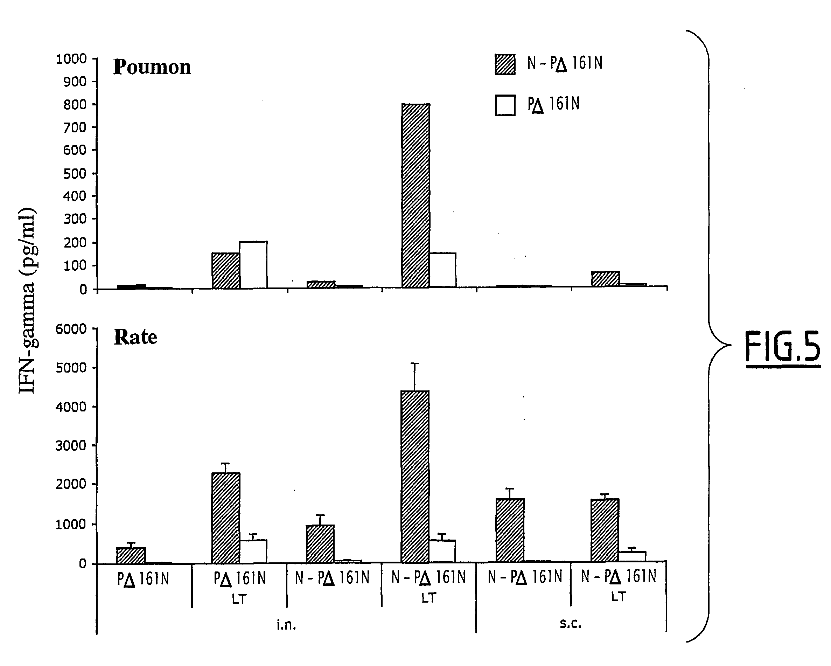

Les réponses les plus fortes sont obtenues après restimulation avec le complexe N-PΔ161N (Figure 5). Comme pour les anticorps, l'administration de N-PΔ161N par voie nasale ou cutanée stimule une immunité cellulaire périphérique (rate) spécifique de N. De façon remarquable, N administrée avec adjuvant par voie nasale génère une immunité cellulaire locale (poumon) (Figure 5).

En conclusion, la protéine N structurée en anneau se révèle très immunogène, y compris pour stimuler une réponse locale (muqueuse respiratoire).

Ici est donc décrite une composition vaccinale comprenant des protéines N solubles d'un virus de la famille des *Paramyxoviridae,* lesdites protéines N solubles étant dans un véhicule pharmaceutiquement acceptable. La présente invention concerne ainsi une composition vaccinale comprenant des protéines N solubles d'un virus de la famille des *Paramyxoviridae,* dans un véhicule pharmaceutiquement acceptable, lesdites protéines N étant sous forme de ribonucléocapsides, et ledit virus de la famille des *Paramyxoviridae* étant un virus de la sous-famille des *Pneumovirinae.* Ces protéines N solubles sont en particulier susceptibles d'être obtenues par un procédé de séparation de protéines N solubles à partir des complexes solubles N - protéine P tronquée, en plus spécifiquement N-PΔN, tel que décrit plus haut.

Comme expliqué plus haut, les protéines N solubles peuvent être utilisées en vaccination sous forme de complexe avec la protéine P sans effet défavorable. En conséquence, une composition vaccinale décrite ici peut comprendre un complexe soluble protéine N - protéine P d'un virus de la famille des *Paramyxoviridae,* dans un véhicule pharmaceutiquement acceptable.

La présente invention concerne donc également une composition vaccinale comprenant un complexe soluble protéine N -protéine P tronquée selon l'invention, dans un véhicule pharmaceutiquement acceptable.

Pour des questions de rendement de production de ces complexes, l'invention porte préférentiellement sur une composition vaccinale comprenant un complexe soluble protéine N - protéine P tronquée d'un virus de la famille des *Paramyxoviridae,* dans un véhicule pharmaceutiquement acceptable, dans laquelle ladite protéine P tronquée ne contient pas le domaine d'oligomérisation P et est capable d'interagir avec la protéine N.

De préférence, la protéine P tronquée comprend ou est un fragment C-terminal de la protéine P.

L'invention concerne ainsi plus particulièrement un complexe soluble protéine N - fragment C-terminal de la protéine P d'un virus de la famille des *Paramyxoviridae,* dans un véhicule pharmaceutiquement acceptable, dans laquelle ledit fragment C-terminal de la protéine P ne contient pas le domaine d'oligomérisation P et est capable d'interagir avec la protéine N.

Ledit virus de la famille des *Paramyxoviridae* peut être un *Paramyxovirinae* ou un *Pneumovirinae.* En particulier, le virus peut être sélectionné dans le groupe constitué du virus des oreillons, du virus de la rougeole et du virus parainfluenza. De préférence, les protéines N solubles d'un virus de la famille des *Paramyxoviridae* sont des protéines N solubles du virus respiratoire syncytial (VRS), par exemple humain ou bovin.

Selon un mode de réalisation, le virus *Paramyxoviridae* est le virus respiratoire syncytial (VRS) et ledit fragment C-terminal de la protéine P est un fragment C-terminal (PΔN) qui comprend les 9 derniers acides aminés C-terminaux de la protéine P du VRS et est dépourvu d'au moins les 119, de préférence, les 149, de préférence encore les 160 acides aminés N-terminaux de la protéine P du VRS.

Plus spécifiquement, dans ladite composition, le fragment C-terminal de la protéine P du VRS peut être :
a) un fragment C-terminal qui comprend la séquence d'acides aminés 233 à 241 de la protéine P du VRS humain souche LONG telle que montrée dans SEQ ID No.1, et qui s'étend en direction N-terminale jusqu'à un résidu d'acide aminé compris entre les positions 233 et 120, de préférence 150, de préférence encore 161, de la séquence de la protéine P du VRS telle que montrée dans SEQ ID No.1, ou
b) un fragment C-terminal, homologue du fragment défini en a), d'une protéine P issue d'une autre souche du VRS humain ou d'une souche du VRS bovin.

Il peut s'agir en particulier d'un fragment C-terminal de la protéine P du VRS sélectionné dans le groupe constitué de PΔ120N, PΔ150N, PΔ161N, PΔ180N, PΔ200N, PΔ220N, PΔ230N et PΔ233N.

L'invention concerne également une composition dans laquelle la protéine P tronquée du VRS comprend un fragment C-terminal de la protéine P du VRS tel que décrit ci-dessus, qui comprend les 9 derniers acides aminés C-terminaux de la protéine P du VRS et est dépourvu d'au moins les 119, de préférence, les 149, de préférence encore les 160 acides aminés N-terminaux de la protéine P du VRS.

Par exemple la protéine P tronquée du VRS qui comprend un fragment C-terminal de la protéine P peut être constituée par la fusion des 122 derniers acides aminés N-terminaux avec les 80 derniers acides aminés C-terminaux de la protéine P du VRS ; elle peut par exemple être constituée par l'enchaînement des acides aminés 1 à 121 et 161 à 241 de la protéine P du VRS humain souche LONG telle que montrée dans SEQ ID No.1.

Selon un exemple exclu de l'invention, le virus *Paramyxoviridae* est le virus de la rougeole et ledit fragment de la protéine P est un fragment C-terminal de la protéine P qui comprend au plus, ou est constitué de, les 122 acides aminés G-terminaux de la protéine P. Plus spécifiquement, ledit fragment C-terminal de la protéine P du virus de la rougeole peut être constitué par les acides 386 à 514 de la protéine P (PΔ386N) du virus de la rougeole souche Edmonston B, telle que montrée dans SEQ ID No.3, ou être un fragment C-terminal, homologue à celui défini pour protéine P de la souche Edmonston, d'une protéine P issue d'une autre souche de virus de la rougeole.

Par « véhicule pharmaceutiquement acceptable », on entend tout solvant, milieu de dispersion, agents retardant l'absorption etc, qui ne produisent pas de réaction secondaire, par exemple allergique, chez l'humain ou l'animal.

Avantageusement, la composition vaccinale selon l'invention peut en outre comprendre un adjuvant. Un « adjuvant » désigne un produit qui augmente, stimule, active, renforce, ou module la réaction immunitaire au niveau cellulaire ou humoral dirigée contre un antigène administré simultanément. Des exemples d'adjuvants classiques incluent des adjuvants contenant des antigènes bactériens, tels que l'adjuvant complet de Freund, le LPS et ses dérivés, les toxines bactériennes (choléra toxine et entérotoxine) et leurs mutants détoxifiés (par exemple LT(R192G)), des séquences oligonucléotidiques contenant des motifs CpG, des adjuvants minéraux tels que l'hydroxyde d'aluminium (Alum), le phosphate de calcium ou le phosphate de potassium, des émulsions huileuses et agents émulsifiants (saponines, par exemple QS21), des cytokines.

Les compositions vaccinales selon l'invention permettent de conférer une protection vis-à-vis d'une infection par un virus de la famille des *Paramyxoviridae*, c'est-à-dire une réduction de la gravité des effets d'une telle infection par rapport à un sujet non immunisé avec la composition vaccinale.

L'invention concerne également l'utilisation d'une composition vaccinale telle que définie plus haut dans une méthode de vaccination.

L'invention porte donc sur une méthode de vaccination comprenant au moins une administration d'une composition vaccinale selon l'invention à un sujet. De préférence, la méthode de vaccination comprend une première administration à un sujet d'une composition vaccinale, et une administration de rappel de ladite composition vaccinale au même sujet. Les administrations de rappel, en réexposant le patient à l'antigène, induisent une réponse immunitaire secondaire plus forte.

L'expression « sujet » désigne un humain ou un animal non-humain, par exemple un oiseau ou une mammifère tel qu'un bovin, un rongeur, un chien, un chat, un porc, un singe, exposé ou susceptible d'être exposé à une infection par un virus *Paramyxoviridae.* Préférentiellement un sujet au sens de l'invention est un humain ou un bovin.

La composition vaccinale est avantageusement administrée en une quantité efficace pour induire une réponse immune protectrice ou thérapeutique vis-à-vis d'une infection par un virus de la famille des *Paramyxoviridae.* La posologie dépend naturellement de l'actif considéré, du mode d'administration, de l'âge du sujet et de son état. La quantité de complexe N-P, N-ΔPN, ou de protéine N par dose peut être comprise entre 0,1 et 200 µg, et préférablement comprise entre 10 et 100 µg par dose vaccinale.

La composition vaccinale peut être administrée par n'importe quelle voie, en particulier par voie mucosale (par exemple oculaire, intranasale, orale), ou par voie parentérale (par exemple sous-cutanée, intradermique, intramusculaire, intraveineuse, ou intrapéritonéale).

### Applications diagnostiques

Les protéines N solubles d'un virus de la famille des *Paramyxoviridae,* éventuellement sous forme de complexe soluble protéine N - protéine P tronquée, constituent par ailleurs un réactif susceptible d'être utilisé dans des applications diagnostiques pour la détection d'anticorps dirigés contre ladite protéine N du virus *Paramyxoviridae.*

Est donc en outre ici décrit un réactif diagnostic comprenant une protéine N d'un virus de la famille des *Paramyxoviridae,* éventuellement sous forme de complexe protéine N-protéine P, telle que décrite plus haut.

La présente invention concerne ainsi en outre un réactif diagnostic comprenant une protéine N d'un virus de la famille des *Paramyxoviridae,* dans lequel ladite protéine N est sous la forme d'un complexe protéine N - protéine P tronquée du même virus de la famille des *Paramyxoviridae* selon l'invention, ledit virus de la famille des *Paramyxoviridae* étant un virus de la sous-famille des *Pneumovirinae.*

La présente invention concerne également un réactif diagnostic comprenant une protéine N du VRS soluble selon l'invention.

Un kit diagnostic comprenant ledit réactif et des moyens de détection appropriés est également dans la portée de l'invention.

Est ici également décrite l'utilisation d'une protéine N d'un virus de la famille des *Paramyxoviridae* selon l'invention, pour la détection, *in vitro* ou *in vivo,* d'anticorps dirigés contre ladite protéine N.

La présente invention concerne également l'utilisation d'une protéine N d'un virus de la famille des *Paramyxoviridae,* ladite protéine N soluble étant sous la forme d'un complexe protéine N - protéine P tronquée du même virus de la famille des *Paramyxoviridae* selon l'invention, ledit virus de la famille des *Paramyxoviridae* étant un virus de la sous-famille des *Pneumovirinae* pour la détection, in vitro, d'anticorps dirigés contre ladite protéine N.

La présente invention a également pour objet l'utilisation d'une protéine N du VRS selon l'invention, pour la détection, in vitro, d'anticorps dirigés contre ladite protéine.

Est aussi décrite une méthode de détection, dans un échantillon biologique, d'anticorps dirigés spécifiquement contre la protéine N d'un virus de la famille des *Paramyxoviridae,* ladite méthode comprenant les étapes consistant à :
a) mettre en contact ledit échantillon biologique avec une protéine N d'un virus de la famille des *Paramyxoviridae,*
b) détecter les complexes protéine N/anticorps formés,
la présence de tels complexes étant révélatrice de la présence d'anticorps spécifiques de la protéine N du virus de la famille des *Paramyxoviridae* dans l'échantillon biologique.

La présente invention a également pour objet une méthode de détection, dans un échantillon biologique, d'anticorps dirigés spécifiquement contre la protéine N d'un virus de la famille des *Paramyxoviridae,* ladite méthode comprenant les étapes consistant à :
a) mettre en contact ledit échantillon biologique avec une protéine N d'un virus de la famille des *Paramyxoviridae,* ladite protéine N ayant la forme d'un complexe protéine N - protéine P tronquée du même virus de la famille des *Paramyxoviridae* selon l'invention,
b) détecter les complexes protéine N/anticorps formés,
la présence de tels complexes étant révélatrice de la présence d'anticorps spécifiques de la protéine N du virus de la famille des *Paramyxoviridae* dans l'échantillon biologique,
dans laquelle ledit virus de la famille des *Paramyxoviridae* est un virus de la sous-famille des *Pneumovirinae*

L'échantillon biologique peut être un échantillon de tissu obtenu par exemple par biopsie musculaire, hépatique, cardiaque, cérébrale, etc, ou un échantillon liquide, par exemple un liquide biologique tel que le sang, le plasma, ou le liquide céphalorachidien.

La détection des complexes peut être réalisée par des moyens usuels bien connus de l'homme du métier tels que la chromatographie (d'exclusion de taille, d'affinité, etc) ou l'électrophorèse en conditions non dénaturantes.

Dans la méthode de détection d'anticorps dirigés spécifiquement contre la protéine N telle que définie ci-dessus, la protéine N mise en contact avec l'échantillon biologique peut avoir la forme d'un complexe protéine N - protéine P.

Les exemples et figures suivantes illustrent l'invention sans en limiter sa portée.

### FIGURES

La Figure 1 montre la cartographie du domaine d'interaction P-N sur P. La protéine P a été fusionnée à la GST et co-exprimée chez E. coli avec la protéine N exprimée sur un autre plasmide.
La Figure 2 montre le profil d'élution des complexes N-PΔ161N en chromatographie d'exclusion de taille. (A) Profil d'élution à 220 nm en colonne TSK. (B) Analyse par électrophorèse en gel d'acrylamide des différentes fractions après coloration au bleu de Coomassie. Les fractions 17 à 22 ne contiennent que des anneaux ARN-N.
La Figure 3 montre la structure des anneaux de protéine N du VRS. (A) Analyse au microscope électronique des anneaux N-ARN purifiés par P161-241. (B) Reconstruction par cryomicroscopie. (C) Analyse de l'ARN présent dans les anneaux en gel d'agarose.
La Figure 4 décrit les résultats de l'analyse de l'immunogénicité de la protéine N structurée en anneau par mesure de la production d'anticorps dirigés contre le complexe N-PΔ161N. Des souris BALB/c ont été immunisées par voie intra-nasale (i.n.) ou sous-cutanée (s.c.) avec 20 µg de complexe N-P161ΔN ou PΔ161N en présence ou non de l'adjuvant muqueux LT(R192G). Un rappel est réalisé après deux semaines (J14). Les animaux sont euthanasiés deux semaines après le rappel (J28). Pour la mesure des anticorps sériques, le sérum est collecté à J0, J14 et J28 (A). Pour la mesure des anticorps muqueux, les lavages broncho-alvéolaires sont réalisés à J28 (B). Le taux d'anticorps contre N-P161ΔN est mesuré par ELISA. Les données sont exprimées comme la moyenne ± erreur standard à la moyenne (n = 5) et représentées avec une échelle logarithmique.
La Figure 5 décrit les résultats de l'analyse de l'immunogénicité de la protéine N structurée en anneau par mesure de la réponse cellulaire spécifique de PΔ161N et N-PΔ161N. Des souris BALB/c ont été immunisées par voie intra-nasale (i.n.) ou sous-cutanée (s.c.) avec 20 µg de complexe N-PΔ161N ou de PΔ161N en présence ou non de l'adjuvant muqueux LT(R192G). Un rappel est réalisé après deux semaines (J14). Les animaux sont euthanasiés deux semaines après le rappel (J28) pour prélever rates et poumons. Les cellules de la rate et du poumon sont mises en culture pendant 72h en présence de N-PΔ161N, PΔ161N ou sans restimulation. La sécrétion d'IFN-γ est mesurée par ELISA. Sans restimulation, le niveau basal d'IFN-γ est inférieur à 15 pg/ml. Les données sont exprimées comme la moyenne ± erreur standard à la moyenne (n = 5).

### EXEMPLES

### Exemple 1 : Construction des plasmides contenant la région C-terminale de la phosphoprotéine du VRS.

La protéine P du VRS souche Long est composée de 241 résidus d'acides aminés.

Séquences des amorces oligonucléotidiques (de 5' vers 3') utilisées pour amplifier la partie C-terminale de la protéine P du VRS (les sites de restriction BamHI sont soulignés ; le codon « start » ATG du gène P est figuré en caractères gras) :
LONG-PBam+ : GAGGGATCCATC**ATG**GAAAAGTTTGCTCCTG (SEQ ID No.11)
LONG-P- : CTGTTGGTGTTGTGTGTTGAAGTGCAG (SEQ ID No. 12)
P161B+: GAGGGATCCTCTGCTAGGGATGGTATAAGAG (SEQ ID No. 13)
P180B+ : GAGGGATCCAAAATCAGAACTGAAGCATTAATGACC (SEQ ID No.14)
P201B+: GAGGGATCCGAGGAAAGTGAAAAGATGGCAAAAG (SEQ ID No.15)
P221B+: GAGGGATCCGAGAAATTGAACAACCTGTTGG (SEQ ID No.16)
P230NB+ : GATCCAATGATAGTGACAATGATCTATCACTTGAAGATTTCTGA (SEQ ID No.17)
P230N-: TCAGAAATCTTCAAGTGATAGATCATTGTCACTATCATTG (SEQ ID No.18)

L'ADNc du gène P du VRS souche Long a été amplifié par RT-PCR à partir de cellules Hep-2 infectées par la souche Long du VRS humain en utilisant les amorces LONG-PBam+ et LONG-P- (Castagné et al., 2004). Le produit de PCR a été digéré par l'enzyme de restriction BamHI et cloné dans le plasmide pGEX-4T-3 (Pharmacia) aux sites BamHI-Smal en phase avec le gène codant pour la Glutathion-S-Transferase ou GST. Le plasmide s'appelle pGEX-P.

### • Clonage de P161-241 (PΔ161N)

La région C-terminale de P (acides aminés 161-241) a été amplifiée par PCR à partir du plasmide pGEX-P dans les conditions suivantes :
Amorces de PCR : P161B+ et LONG-P-100 ng chaque (1µl chaque)
ADN matrice pGEX-P : 10 ng (1µl)
Enzyme : Pfu Turbo marque Stratagene (unités par µl) : 1 µl
dATP : 0.2 mM final
dGTP : 0.2 mM final
dCTP : 0.2 mM final
dTTP : 0.2 mM final
Tampon Pfu 1X final (Stratagene)
Volume final : 100 µl

La PCR a été réalisée dans les conditions suivantes :
5 cycles : 15 secondes à 94°C, 2 minutes à 40°C, 1 minute à 72°C ;
25 cycles : 15 secondes à 94°C, 1 minutes à 55°C, 1 minute à 72°C

L'ADN amplifié a été extrait par un volume (100 µl) de phénol/chloroforme (1vol/1vol), puis par un volume de chloroforme, enfin précipité par ajout d'un dixième de volume de NaCl 5M (10 µl) et deux volumes d'éthanol à 100% (200 µl). L'ADN a été centrifugé 20 minutes à 13000 g, lavé avec un volume d'éthanol à 70%, séché, resuspendu dans un volume d'eau de 90 µl. Après ajout de 10 µl de tampon 10X pour l'enzyme BamHI, l'ADN a été digéré 2 heures à 37°C en présence de 10 unités d'enzyme BamHI. L'ADN digéré a été déposé sur un gel d'agarose à 1,5 % dans du tampon Tris-Borate-EDTA (TBE) 1X en présence de bromure d'éthidium et mis à migrer par électrophorèse. La bande correspondant à l'ADN de P161-241 a été découpée et l'ADN extrait par électroélution. Il a été à nouveau extrait avec un volume de phénol-chloroforme, un volume de chloroforme et précipité à l'éthanol. Il a été ligué avec le vecteur pGEX-4T-3 digéré par BamHI et SmaI après purification en gel d'agarose à 1 % :
ADN pGEX4T-3 : 100ng
ADN P161-241 : 100 ng
Tampon ligase 1X final
Ligase (5U/µl) : 1 µl
Volume final 20 µl

L'ensemble a été incubé une nuit à 14°C. Le lendemain des bactéries DH5-alpha TM (Life Technologies) compétentes ont été transformées avec 10 µl de produit de ligation et étalées sur boîte de Pétri contenant du milieu L-agar supplémenté avec 100 µg/ml d'ampicilline final. Les colonies de bactéries recombinantes ont été criblées par mini préparation de plasmide et digestion par les enzymes de restriction BamHI et XhoI. Les plasmides recombinants montrent alors deux bandes en gel d'agarose, une correspondant au vecteur (4,9 kb) et la seconde correspondant à la partie C-terminale de P (246 pb). Les plasmides recombinants ont été entièrement séquencés.

### • Clonage de P180-241. P201-241, P221-241

Les fragments de P correspondants aux parties d'acides aminés 180-241, 200-241, 220-241 ont été obtenus par PCR à partir du plasmide pGEX-P en utilisant les amorces suivantes :
P180-241 : amorces P180B+ et LONG-P
P200-241 : amorces P201 B+ et LONG-P-
P220-241 : amorces P221B+ et LONG-P-

Ils ont été amplifiés et clonés de la même façon que P161-241 (voir au-dessus).

### • Clonage du gène codant pour la protéine de nucléocapside du VRS souche Long.

Le gène codant pour la protéine N du VRS humain souche Long a été obtenu par RT-PCR à partir de cellules Hep-2 infectées par du virus. Les amorces utilsées étaient :
LONG-Nbam+ : GAGGGATCCATGGCTCTTAGCAAAGTCAAGTTG (SEQ ID No.19)
LONG-N- TTAACTCAAAGCTCTACATCATTATCTTTTGG (SEQ ID No.20)

Les produits de PCR ont été digérés par BamHI et clonés dans le plasmide pGEX-4T-3 aux sites BamHI-SmaI. La région codant pour N (SEQ ID No.) a été sous-clonée par digestion du plasmide pGEX-N par BamHI-XhoI et sous-clonée dans le plasmide pET28a+ (Novagen ; SEQ ID No. et voir Figure).

### • Clonage de P231-241

Les amorces suivantes ont été dénaturées par chauffage à 94°C, pendant 5 minutes, refroidies à température ambiante :
P231NB+ GATCCGATAGTGACAATGATCTATCACTTGAAGATTTCTGA (SEQ ID No.21)
P231 N- TCAGAAATCTTCAAGTGATAGATCATTGTCACTATCG (SEQ ID No.22)

Après hybridation, 10 ng d'oligonucléotides double brin ont été ligués avec 100 ng d'ADN de plasmide pGEX-4T-3 digéré par les enzymes BamHI et Smal et purifié par électrophorèse en gel d'agarose. Les plasmides recombinants ont été vérifiés par séquençage au niveau du gène N.

### Exemple 2 : Expression et purification des complexes

Les bactéries BL21 (DE3) (Novagen) compétentes ont été transformées avec 1 µg d'ADN pGEX-PΔ et 1 µg d'ADN pET-N puis étalées sur boîte de Pétri contenant du milieu L-agar supplémenté avec 100 µg/ml final d'ampicilline et 50 µg/ml final de Kanamycine. Une colonie a été repiquée et mise à pousser une nuit à 37°C dans 2 ml de milieu LB contenant Ampicilline et Kanamycine à 100 µg/ml et 50 µg/ml respectivement. Le lendemain matin 1 ml de culture saturée a servi à ensemencer 1 litre de milieu LB additionné des antibiotiques et mis à pousser jusqu'au soir. Le soir, un volume de milieu frais LB contenat de l'IPTG (qui induit l'expression des protéines) à une concentration de 160 µg/ml est ajouté à la culture et le tout est mis à pousser sur la nuit à 28°C. Le lendemain, les bactéries sont centrifugées 15 minutes à 5000 rpm et le culot est repris dans 100 ml du tampon suivant :
50 mM Tris pH 7,8
60 mM NaCl
2 mM DTT
1 mM EDTA
4 mM Benzamidine
Antiprotéases 1X (Complete EDTA-free protease inhibitor cocktail, ref. Roche n° 11 873 580 001) soit une tablette pour 50 ml de tampon de lyse 0,1% Triton-X100

10 ml du même tampon additionné de Lysozyme à 10mg/ml (1mg/ml final) sont ajoutés. Les bactéries sont incubées 1 heure dans la glace (lyse). Quand le tout devient visqueux, l'ensemble est soniqué 3 fois 1 minute en utilisant une sonde plongeant dans le mélange, dans la glace en laissant reposer 5 minutes entre chaque sonication. L'ensemble est centrifugé 30 minutes à 10 000 g à 4°C puis le surnageant est récupéré. Le surnageant est recentrifugé 30 minutes à 10 000 g à 4°C puis le nouveau surnageant est récupéré. 4 ml de billes de sepharose 4B-glutathion (Amersham-Pharmacia) sont lavés en prélevant 8 ml de mélange billes tampon (vol/vol) avec le tampon de lyse. Les billes sont laissées dans un volume équivalent de tampon, ajoutées au lysat bactérien clarifié, et mises à tourner à 4°C sur la nuit. Le lendemain les billes sont centrifugées à 2000 rpm pendant 3 minutes, puis le surnageant est éliminé, et les billes lavées trois fois avec le tampon de lyse dépourvu d'antiprotéases, trois fois en tampon PBS 1X.

Les billes son clivées au site thrombine en utilisant de la Thrombin biotinylée (Novagen) à raison d'1 µl (1 U) de thrombin (Thrombin Cleavage Capture Kit, N° 69022-3FRZ) pour 1 ml de billes. Les billes sont incubées sur la nuit à 20°C et, le lendemain, centrifugées 3 minutes à 2000 rpm, laissées à décanter 15 minutes pour récupérer le surnageant. Un volume équivalent de PBS 1X est ajouté sur les billes ; le mélange est agité, laissé décanter. Le surnageant est à nouveau récupéré et rajouté au surnageant prélevé précédemment. Au surnageant récupéré sont ajoutées des billes d'agarose Streptavidine (Novagen ref. 69203) à raison de 16 µl de résine (soit 32 µl de mélange résine/tampon (vol/vol). Le mélange est laissé en agitation pendant une heure, puis centrifugé 3 minutes à 2000 rpm et le surnageant est récupéré. On obtient une concentration en protéines de 2 mg/ml.

10 µl du surnageant contenant les produits de clivage sont dénaturés en tampon de Laemmli 1X, mis à bouillir et déposés sur un gel de polyacrylamide à 12% en tampon Tris-Glycine SDS 0,1%, puis colorés au bleu de Coomassie après électrophorèse pour visualiser les protéines.

### Exemple 3 : Séparation de N et PΔ161N (P161-241) et purification des anneaux de N

Les protéines présentes dans le surnageant peuvent être séparées par chromatographie d'exclusion de taille (gel filtration, Figure 2) en PBS 1X. La protéine N est exclue à une taille apparente de 450 000 Da et PΔ161N avec une masse de 15 kDa.

Une observation au microscope électronique de la fraction « N » issue de la chromatographie d'exclusion de taille montre que la protéine N se présente sous forme d'anneaux (Figure 3A) ayant un diamètre de 7 nm et contenant 10 sous-unités de N (Figure 3B). Les anneaux contiennent un ARN d'environ 70 pb (Figure 3C)

### Exemple 4 : Evaluation des propriétés immunogéniques de la protéine N recombinante structurée en anneau

### • Une vaccination par voie nasale ou sous-cutanée est effectuée chez des souris, en présence ou non d'adjuvant :

Souris: 30 souris BALB/c femelles de 10-12 semaines, élevées à l'Unité Expérimentale Animalerie Rongeur (INRA, Jouy-en-Josas).
Antigènes: P161-241 et complexe {P161-241 + N} solubles à une concentration de 1 mg/ml après séparation de la GST par clivage à la thrombine et élimination de la thrombine biotinylée par des billes couplées à la streptavidine.
Adjuvant : LT(R192G) lymphotoxine de E. Coli, 1 mg/ml (Choi et al., 2004).

### Prélèvements

- à J0, J14 et J28, prise de sang dans le sinus rétro-orbital
- à J28 :
   - Lavage broncho-alvéolaire (LBA) avec 1,5 ml de milieu HBSS et EDTA 1 mM.
   - Dissection de la rate et du poumon en milieu RPMI plus antibiotiques (Penicillin 100 UI/ml et Streptomycin 100 µg/ml, PS), sur la glace.

### Plan expérimental :

| Groupes (5 souris) | traitements | J0 primo-injection | J14 rappel | J28 autopsie |
|---|---|---|---|---|
| 1 | Intra-nasale 20 µg de protéines ± 10 µg de LT(R192G), sous un volume de 50 µl | P161-241 | P161-241 | Sérum LBA Rate Poumon |
| 2 | | P161-241 LT(R192G) | P161-241 LT(R192G) | |
| 3 | | P161-241 + N | P161-241 + N | |
| 4 | | P161-241 + N LT(R192G) | P161-241 + N LT(R192G) | |
| 5 | Sous-cutanée 20 µg de protéines ± 10 µg de LT(R192G), sous un volume de 50 µl | P161-241 + N | P161-241 + N | |
| 6 | | P161-241 + N LT(R192G) | P161-241 + N LT(R192G) | |

### • Production d'anticorps anti-N

Les sérums sont collectés à partir des prélèvements de sang (1 nuit d'exsudation à 4°C) puis congelés à -20°C.

Les LBA sont centrifugés 5 min à 1700 rpm, les surnageant sont collectés (environ 1 ml) et congelés à -20°C.

Les anticorps anti-N (Ig totales, IgG1, IgG2a et IgA) sont recherchés dans les sérums et les LBA par E.L.I.S.A : des plaques de 96 puits (Immulon 2HB, ThermoLabsystems) sont sensibilisées une nuit à 4°C avec le complexe P161-241+N (200 ng par puit) en tampon bicarbonate 0,1 M pH 9,5. Les plaques sont lavées 5 fois avec 200 µl par puit de PBS 0,05 % tween 20 (utilisation d'un automate Wellwash, Labsystems). Les plaques sont ensuite saturées pendant 1 h à 37°C avec 150 µl par puit de tampon PBS 0,05 % tween 20 et 5 % sérum de veau foetal (PBS-T-SVF). Après 5 lavages, les échantillons à titrer sont dilués en PBS-T-SVF (sept dilutions successives de raison 3 en partant d'une première dilution au 30^{ème} pour les sérums et au tiers pour les LBA). Les plaques sont incubées pendant 2 h à 37°C. Après 5 lavages, l'anticorps secondaire dilué en PBS-T-SVF est distribué à raison de 100 µl par puit. Les anticorps secondaires utilisés sont conjugués à la péroxydase et dirigés contre les immunoglobulines de souris : IgG totales (4000^{ème}, P.A.R.I.S.), IgG1 (2000^{ème}, BD biosciences), IgG2a (2000^{ème}, BD biosciences) ou IgA (1000^{ème}, Caltag). Les plaques sont incubées pendant 2 h à 37°C et lavées 5 fois. Les plaques sont ensuite incubées avec le substrat de la péroxydase (TMB, 100 µl par puit) pendant 10 min à l'obscurité. La réaction enzymatique est arrêtée par l'ajout de 50 µl de H₃PO₄ 2M. Les densités optiques (DO) sont lues à 450 nm (lecteur Dynex). La courbe D0₄₅₀=f(dilution) est modélisée par la courbe de régression y=(b+cx)/(1+ax). Le titre en anticorps est déterminé comme la valeur de dilution donnant deux fois la DO₄₅₀ d'un échantillon témoin (J0) à sa plus grande dilution.

### • Production d'IFN-γ par des lymphocytes T spécifigues de P161-241 et N

Les rates et les poumons prélevés sont traités selon le même protocole. Les rates sont traitées individuellement et les poumons sont groupés par lot expérimental (5 poumons par lot).

Les tissus sont émincés puis broyés délicatement sur filtre (tamis cellulaire 100 µm, BD Falcon) en milieu RPMI et PS. La suspension cellulaire est centrifugée à 1700 rpm pendant 10 min à 4°C. Les cellules sont remises en suspension dans 1 ml de tampon de lyse des érythrocytes (tampon salin hypotonique) et incubées pendant 5 min à température ambiante. La réaction de lyse est arrêtée par ajout de 10 ml de RPMI complet (PS, 2 mM L-glutamine et 10 % SVF). Les débris membranaires sont décantés et les cellules sont lavées trois fois par centrifugation (1700 rpm pendant 10 min à 4°C). Les suspensions cellulaires sont dénombrées à l'aide d'une cellule de Malassez.

Les cellules sont mises en culture dans des microplaques de 96 puits traitées pour la culture cellulaire (Falcon) à raison de 200000 cellules par puit dans 200 µl de milieu RPMI complet.

Quatre conditions de culture sont testées en triplicat sur chaque suspension cellulaire :
- PMA (Phorbol 12-myristate 13-acétate, Sigma) 10 ng/ml et ionomycine (Sigma) 1 µg/ml (témoin positif, activation polyclonale)
- RPMI complet (témoin négatif)
- P161-241 10 µg/ml
- P161-241+N 10 µg/ml

Après 72h de culture à 37°C avec 5% CO₂, les surnageants de culture sont collectés et congelés à -20°C jusqu'à titration de l'IFN-γ par ELISA.

ELISA IFN-γ : des plaques de 96 puits (Immulon 2HB, ThermoLabsystems) sont sensibilisées une nuit à 4°C avec l'anticorps de capture anti-IFN-γ de souris (BD Bioscience) à 4 µg/ml en tampon Bicarbonate 0,1 M pH 9,5 (100 µl/puit). Les plaques sont lavées 5 fois avec 200 µl par puit de PBS 0,05% tween 20 (utilisation d'un automate Wellwash, Labsystems). Les plaques sont ensuite saturées pendant 2 h à 37°C avec 150 µl par puit de tampon PBS 0,05 % tween 20 et 2 % d'albumine sérique bovine (PBS-T-BSA). Après 5 lavages, la référence IFN-γ de souris recombinant (R&D systems) et les échantillons à titrer sont dilués en PBS-T-BSA par dilutions successives au demi. La gamme IFN-γ est diluée de 3312,5 µg/ml à 3,235 pg/ml. Quatre dilutions successives au demi sont réalisées sur les échantillons purs. La plaque est alors incubée pendant une nuit à 4°C. Après 5 lavages, l'anticorps de détection biotinylé (BD Biosciences) est distribué (1 µg/ml en PBS-T-BSA, 100 µl/puit) et incubé 3 h à 4°C. Après 5 lavages, le conjugué Streptavidine-Péroxydase (Pierce) est distribué (1 µg/ml en PBS-T-BSA, 100 µl/puit) et incubé 1 h à 4°C. Après 5 lavages, le substrat de la péroxydase (ABTS+H₂O₂) est distribué dans les puits. Après 45 minutes d'incubation, les densités optiques sont lues à 405 nm (Lecteur ELISA Dynex). Le calcul de la concentration en IFN-γ dans les échantillons se fait par rapport à la gamme d'IFN-γ.

### RÉFÉRENCES

Ames, T. R. 1993. The epidemiology of BRSV infection. Vet. Med. 881-884.
Bhella, D., Ralph, A., Murphy L. B., & Yeo, R. P. 2002. Significant différences in nucleocapsid morphology within the Paramyxoviridae. Journal of General Virology ; 83, 1831- 1839.
Castagné, N., A. Barbier, J. Bernard, H. Rezaei, J.-C. Huet, C. Henry, B. Da Costa, and J.-F. Eléouët. 2004. Biochemical characterization of the Respiratory Syncytial Virus P-P and P-N protein complexes and localization of the P protein oligomerization domain. Journal of General Virology ; 85 : 1643-1653.
Choi et al., 2004, Protein Expression and Purification ; 38, pp205
Elvander, M. 1996. Severe respiratory disease in dairy cows caused by infection with bovine respiratory syncytial virus. Vet. Rec. ; 138, 101-105.
Freytag, LC et Clements, JD. 2005. Mucosal adjuvants. Vaccine. ; 23(15):1804-13.
Gaddum, R. M., R. S. Cook, J. M. Furze, S. A. Ellis & G. Taylor. 2003. Récognition of bovine respiratory syncytial virus proteins by bovine CD8a T lymphocytes. Immunology ; 108, 220-229;
Goulder PJ, Lechner F, Klenerman P, Mclntosh K, Walker BD. 2000. Characterization of a novel respiratory syncytial virus-specific human cytotoxic T-lymphocyte epitope. J Virol.; 74(16):7694-7.
Johansson et al., 2003 ; Journal of Biological Chemistry vol. 278 p 44567-44573.
Khattar SK, Yunus AS, Samal SK. 2001a. Mapping the domains on the phosphoprotein of bovine respiratory syncytial virus required for N-P and P-L interactions using a minigenome system. J Gen Virol.; 82(Pt 4):775-9.
Khattar SK, Yunus AS, Collins PL, Samal SK.. 2001 b. Deletion and substitution analysis defines régions and residues within the phosphoprotein of bovine respiratory syncytial virus that affect transcription, RNA replication, and interaction with the nucleoprotein. Virology. ; 285(2):253-69.
Kolakofsky D, Le Mercier P, Iseni F, Garcin D. 2004. Viral DNA polymerase scanning and the gymnastics of Sendai virus RNA synthesis. Virology. ; 318(2):463-73. Review.
Maggon K, Barik S. 2004. New drugs and treatment for respiratory syncytial virus. Rev Med Virol. 14(3):149-68. Review.
Mallipeddi SK, Lupiani B, Samal SK. 1996. Mapping the domains on the phosphoprotein of bovine respiratory syncytial virus required for N-P interaction using a two-hybrid system. J Gen Virol. ; 77 ( Pt 5):1019-23.
Martinez X, Li X, Kovarik J, Klein M, Lambert PH, Siegrist CA. 1999. Combining DNA and protein vaccines for early life immunization against respiratory syncytial virus in mice. Eur J Immunol. ; 29(10):3390-400.
Mavrakis M, Iseni F, Mazza C, Schoehn G, Ebel C, Gentzel M, Franz T, Ruigrok RW. 2003. Isolation and characterisation of the rabies virus N °-P complex produced in insect cells. Virology.; 305(2):406-14.
McNeal MM, VanCott JL, Choi AH, Basu M, Flint JA, Stone SC, Clements JD, Ward RL. 2002. CD4 T cells are the only lymphocytes needed to protect mice against rotavirus shedding after intranasal immunization with a chimeric VP6 protein and the adjuvant LT(R192G). J Virol. ; 76(2):560-8.
Meric C, Spehner D, Mazarin V. 1994. Respiratory syncytial virus nucleocapsid protein (N) expressed in insect cells forms nucleocapsid-like structures.Virus Res. 31(2):187-201.
Perrin, B., Dannacher, G., et Solsona, M. 1979. Mise en évidence des anticorps contre le virus respiratoire syncytial chez les bovins français. Rec. Med. Vet. 155, 465-471.
Samal SK, Pastey MK, McPhillips TH, Mohanty SB. 1993. Bovine respiratory syncytial virus nucleocapsid protein expressed in insect cells specifically interacts with the phosphoprotein and the M2 protein. Virology. ; 193(1):470-3.
Slack MS, Easton AJ. 1998. Characterization of the interaction of the human respiratory syncytial virus phosphoprotein and nucleocapsid protein using the two-hybrid system. Virus Res.; 55(2):167-76.
Sparer TE, Matthews S, Hussell T, Rae AJ, Garcia-Barreno B, Melero JA, Openshaw PJ. 1998. Eliminating a region of respiratory syncytial virus attachment protein allows induction of protective immunity without vaccine-enhanced lung eosinophilia. J Exp Med ; 187 (11): 1921-6.
Tarbouriech, N., Curran, J., Ruigrok, R. W., & Burmeister, W. P. (2000). Tetrameric coiled coil domain of Sendai virus phosphoprotein. Nature Structural Biology 7, 777-781.
Taylor G, Bruce C, Barbet AF, Wyld SG, Thomas LH. 2005. DNA vaccination against respiratory syncytial virus in young calves. Vaccine ; 23(10):1242-50
Taylor, G. L. H. Thomas, J. M. Furze, R. S. Cook, S. G. Wyld, R. Lerch, R. Hardy and G.W. Wertz. 1997. Recombinant vaccinia viruses expressing the F, G or N, but not the M2, protein of bovine respiratory syncytial virus (BRSV) induce résistance to BRSV challenge in the calf and protect against the development of pneumonie lesions. Journal of General Virology ; 78, 3195-3206.
Thompson W.W., D.K. Shay, E. Weintraub, L. Brammer, N. Cox, L.J. Anderson, K. Fukuda. 2003. Mortality associated with influenza and respiratory syncytial virus in the United States. JAMA. ; 289(2): 179-86.
Wellemans, G., and J. Leunen. 1975. Le virus respiratoire syncytial et les troubles respiratoires des bovins. Ann. Med. Vet. ; 119, 359-369.

## Revendications

1. Procédé de préparation d'un complexe soluble protéine N -protéine P tronquée d'un virus de la famille des *Paramyxoviridae*, ledit procédé comprenant les étapes consistant à :
a) coexprimer une protéine N d'un virus de la famille des *Paramyxoviridae* avec une protéine P tronquée du même virus de la famille des *Paramyxoviridae*, ladite protéine P tronquée ne contenant pas le domaine d'oligomérisation P et étant capable d'interagir avec la protéine N ;
b) recueillir les complexes solubles protéine N -protéine P tronquée ainsi formés;
dans lequel le virus de la famille des *Paramyxoviridae* est un virus de la sous-famille des *Pneumovirinae*.

2. Procédé selon la revendication 1, dans lequel le virus *Paramyxoviridae* est le virus respiratoire syncytial (VRS) humain ou bovin.

3. Procédé selon la revendication 2, dans lequel on coexprime :
a) un fragment C-terminal de la protéine P du VRS qui comprend la séquence d'acides aminés 233 à 241 de la protéine P du VRS humain souche LONG telle que montrée dans SEQ ID No.1, et qui s'étend en direction N-terminale jusqu'à un résidu d'acide aminé compris entre les positions 233 et 161 de la séquence de la protéine P du VRS telle que montrée dans SEQ ID No.1, ou
b) un fragment C-terminal, homologue du fragment défini en a), d'une protéine P issue d'une autre souche du VRS humain ou d'une souche du VRS bovin, le fragment C-terminal de la protéine P étant un enchaînement d'acides aminés positionnés dans la moitié C-terminale de la séquence primaire de la protéine P, ne contenant pas le domaine d'oligomérisation P mais étant capable d'interagir avec la protéine N.

4. Complexe isolé soluble protéine N -protéine P tronquée d'un virus de la famille des *Paramyxoviridae,* ladite protéine N étant sous forme de ribonucléocapside et ledit virus de la famille des *Paramyxoviridae* étant un virus de la sous-famille des *Pneumovirinae,* ladite protéine P tronquée ne contenant pas le domaine d'oligomérisation P et étant capable d'interagir avec la protéine N.

5. Complexe isolé soluble selon la revendication 4, dans lequel la protéine P tronquée est
a) un fragment C-terminal qui comprend la séquence d'acides aminés 233 à 241 de la protéine P du VRS humain souche LONG telle que montrée dans SEQ ID No.1, et qui s'étend en direction N-terminale jusqu'à un résidu d'acide aminé compris entre les positions 233 et 161 de la séquence de la protéine P du VRS telle que montrée dans SEQ ID No.1, ou
b) un fragment C-terminal, homologue du fragment défini en a), d'une protéine P issue d'une autre souche du VRS humain ou d'une souche du VRS bovin, le fragment C-terminal de la protéine P étant un enchaînement d'acides aminés positionnés dans la moitié C-terminale de la séquence primaire de la protéine P, ne contenant pas le domaine d'oligomérisation P mais étant capable d'interagir avec la protéine N.

6. Complexe isolé soluble protéine N - fragment C-terminal de la protéine P du virus respiratoire syncytial (VRS), comprenant 10 molécules de protéine N, et dans lequel ledit fragment C-terminal de P comprend les 9 derniers acides aminés C-terminaux de la protéine P du VRS et est dépourvu d'au moins les 160 acides aminés N-terminaux de la protéine P du VRS.

7. Procédé de préparation de protéines N solubles d'un virus de la famille des *Paramyxoviridae,* ledit procédé comprenant les étapes consistant à :
a) préparer un complexe soluble protéine N -protéine P tronquée par un procédé selon l'une quelconque des revendications 1 à 3 ; et
b) séparer les protéines N solubles des complexes solubles protéine N-protéine P tronquée ;
dans lequel le virus de la famille des *Paramyxoviridae* est un virus de la sous-famille des *Pneumovirinae.*

8. Procédé de préparation de protéines N solubles selon la revendication 7, dans lequel ladite protéine P tronquée est un fragment C-terminal de la protéine P, le fragment C-terminal de la protéine P étant un enchaînement d'acides aminés positionnés dans la moitié C-terminale de la séquence primaire de la protéine P, ne contenant pas le domaine d'o!igomérisation P mais étant capable d'interagir avec la protéine N.

9. Ribonucléocapsides solubles comprenant des protéines N du VRS associées sous forme d'anneaux ayant un diamètre d'environ 7 nm et contenant 10 sous-unités et contenant en outre un ARN d'environ 70 bases.

10. Composition vaccinale comprenant des protéines N solubles d'un virus de la famille des *Paramyxoviridae*, dans un véhicule pharmaceutiquement acceptable, lesdites protéines N étant sous forme de ribonucléocapsides, et ledit virus de la famille des *Paramyxoviridae* étant un virus de la sous-famille des *Pneumovirinae.*

11. Composition vaccinale selon la revendication 10, dans laquelle les protéines N solubles d'un virus de la famille des *Paramyxoviridae* sont susceptibles d'être obtenues par un procédé selon la revendication 7 ou 8.

12. Composition vaccinale comprenant un complexe soluble protéine N -protéine P tronquée selon l'une quelconque des revendications 4 à 6 dans un véhicule pharmaceutiquement acceptable.

13. Réactif diagnostic comprenant une protéine N d'un virus de la famille des *Paramyxoviridae,* dans lequel ladite protéine N est sous la forme d'un complexe protéine N - protéine P tronquée du même virus de la famille des *Paramyxoviridae* tel que défini dans l'une quelconque des revendications 4 à 6, ledit virus de la famille des *Paramyxoviridae* étant un virus de la sous-famille des *Pneumovirinae,* ou une protéine N du VRS selon la revendication 9.

14. Utilisation d'une protéine N d'un virus de la famille des *Paramyxoviridae,* ladite protéine N soluble étant sous la forme d'un complexe protéine N - protéine P tronquée du même virus de la famille des *Paramyxoviridae* tel que défini dans l'une quelconque des revendications 4 à 6, ledit virus de la famille des *Paramyxoviridae* étant un virus de la sous-famille des *Pneumovirinae,* ou d'une protéine N du VRS selon la revendication 9, pour la détection, in vitro, d'anticorps dirigés contre ladite protéine N.

15. Méthode de détection, dans un échantillon biologique, d'anticorps dirigés spécifiquement contre la protéine N d'un virus de la famille des *Paramyxoviridae,* ladite méthode comprenant les étapes consistant à :
a) mettre en contact ledit échantillon biologique avec une protéine N d'un virus de la famille des *Paramyxoviridae,* ladite protéine N ayant la forme d'un complexe protéine N - protéine P tronquée du même virus de la famille des *Paramyxoviridae* tel que défini dans l'une quelconque des revendications 4 à 6,
b) détecter les complexes protéine N/anticorps formés,
la présence de tels complexes étant révélatrice de la présence d'anticorps spécifiques de la protéine N du virus de la famille des *Paramyxoviridae* dans l'échantillon biologique,
dans laquelle ledit virus de la famille des *Paramyxoviridae* est un virus de la sous-famille des *Pneumovirinae*.

## Patentansprüche

1. Verfahren zur Herstellung eines löslichen Protein N - geschnittenen Protein P -Komplexes eines Virus der Familie *Paramyxoviridae*, wobei das Verfahren die Schritte umfasst, bestehend aus:
a) Co-exprimieren eines Proteins N eines Virus der Familie der *Paramyxoviridae* mit einem geschnittenen Protein P des gleichen Virus der Familie der *Paramyxoviridae*, wobei das geschnittene Protein P nicht die Oligomerisationsdomäne P enthält und in der Lage ist, mit dem Protein N zu interagieren;
b) Auffangen der so gebildeten löslichen Protein N - geschnittenen Protein P -Komplexe;
wobei das Virus der Familie der *Paramyxoviridae* ein Virus der Unterfamilie der *Pneumovirinae* ist.

2. Verfahren gemäß Anspruch 1, wobei das Virus *Paramyxoviridae* das humane oder bovine respiratorische Synzytial-Virus (RSV) ist.

3. Verfahren gemäß Anspruch 1, wobei man:
a) ein C-terminales Fragment des Proteins P des RSV, das die Aminosäuresequenz 233 bis 241 des Proteins P des humanen RSV Stamms LONG wie in SEQ ID NR. 1 gezeigt umfasst und das sich in N-terminaler Richtung bis zu einer Aminosäure erstreckt, die zwischen den Positionen 233 bis 161 der Sequenz des Proteins P des RSV wie in SEQ ID Nr. 1 gezeigt umfasst ist, oder
b) ein C-terminales, zu dem in a) definierten Fragment homologes Fragment eines Proteins P, das aus einem anderen Stamm des humanen RSV oder einem Stamm des bovinen RSV stammt, wobei das C-terminale Fragment des Proteins P eine Aneinanderreihung von Aminosäuren ist, die in der C-terminalen Hälfte der Primärsequenz des Proteins P lokalisiert sind, und nicht die Oligomerisationsdomäne P enthält, aber in der Lage ist, mit dem Protein N zu interagieren,
co-exprimiert.

4. Isolierter, löslicher Protein N- geschnittenes Protein P -Komplex eines Virus der Familie der *Paramyxoviridae*, wobei das Protein N in Form eines Ribonucleocapsids ist und das Virus der Familie der *Paramyxoviridae* ein Virus der Unterfamilie der *Pneumovirinae* ist, wobei das geschnittene Protein P nicht die Oligomerisationsdomäne P enthält und in der Lage ist, mit dem Protein N zu interagieren.

5. Isolierter, löslicher Komplex gemäß Anspruch 4, wobei das geschnittene Protein P
a) ein C-terminales Fragment, das die Aminosäuresequenz 233 bis 241 des Proteins P des humanen RSV Stamms LONG wie in SEQ ID NR. 1 gezeigt umfasst und das sich in N-terminaler Richtung bis zu einer Aminosäure erstreckt, die zwischen den Positionen 233 bis 161 der Sequenz des Proteins P des RSV wie in SEQ ID Nr. 1 gezeigt umfasst ist, oder
b) ein C-terminales, zu dem in a) definierten Fragment homologes Fragment eines Proteins P, das aus einem anderen Stamm des humanen RSV oder einem Stamm des bovinen RSV stammt, wobei das C-terminale Fragment des Proteins P eine Aneinanderreihung von Aminosäuren ist, die in der C-terminalen Hälfte der Primärsequenz des Proteins P lokalisiert sind, und nicht die Oligomerisationsdomäne P enthält, aber in der Lage ist, mit dem Protein N zu interagieren,
ist.

6. Isolierter, löslicher Komplex Protein N - C-terminales Fragment des Proteins P eines respiratorischen Synzytial-Virus (RSV), umfassend 10 Moleküle des Proteins N, in dem das C-terminale Fragment von P die 9 letzten C-terminalen Aminosäuren des Proteins P des RSV umfasst und das mindestens die 160 N-terminalen Aminosäuren des Proteins P des RSV ermangelt.

7. Verfahren zur Herstellung von löslichen Proteinen N eines Virus der Familie der *Paramyxoviridae*, wobei das Verfahren die Schritte bestehend aus:
a) Herstellen eines löslichen Protein N- geschnittenen Protein P -Komplexes durch ein Verfahren gemäß einem der Ansprüche 1 bis 3; und
b) Abtrennen der löslichen Proteine N von den löslichen Protein N- geschnittenen Protein P -Komplexen
umfasst;
wobei das Virus der Familie der *Paramyxoviridae* ein Virus der Unterfamilie der *Pneumovirinae* ist.

8. Verfahren zur Herstellung von löslichen Proteinen N gemäß Anspruch 7, wobei das geschnittene Protein P ein C-terminales Fragment des Proteins P ist, wobei das C-terminale Fragment des Proteins P eine Aneinanderreihung von Aminosäuren ist, die in der C-terminalen Hälfte der Primärsequenz des Proteins P lokalisiert sind, und nicht die Oligomerisationsdomäne P enthält, aber in der Lage ist, mit dem Protein N zu interagieren.

9. Lösliche Ribonucleocapside umfassend Proteine N des RSV, die in Form von Ringen mit einem Durchmesser von ungefähr 7 nm verbunden sind und 10 Untereinheiten enthalten und des Weiteren eine RNA von ungefähr 70 Basen enthalten.

10. Impfzusammensetzung umfassend lösliche Proteine N eines Virus der Familie der *Paramyxoviridae* in einem pharmazeutisch annehmbaren Trägerstoff, wobei die Proteine N in Form von Ribonucleocapsiden sind und wobei das Virus der Familie der *Paramyxoviridae* ein Virus der Unterfamilie der *Pneumovirinae* ist.

11. Impfzusammensetzung gemäß Anspruch 10, wobei die löslichen Proteine N eines Virus der Familie der *Paramyxoviridae* durch ein Verfahren gemäß Anspruch 7 oder 8 erhältlich sind.

12. Impfzusammensetzung umfassend einen löslichen Protein N- geschnittenen Protein P -Komplex gemäß einem der Ansprüche 4 bis 6 in einem pharmazeutisch annehmbaren Trägerstoff.

13. Diagnostisches Reagenz umfassend ein Protein N eines Virus der Familie der *Paramyxoviridae*, wobei das Protein N in Form eines Protein N- geschnittenen Protein P-Komplexes des gleichen Virus der Familie der *Paramyxoviridae* wie in einem der Ansprüche 4 bis 6 definiert ist, wobei das Virus der Familie der *Paramyxoviridae* ein Virus der Unterfamilie der *Pneumovirinae* ist, oder ein Protein N des RSV gemäß Anspruch 9.

14. Verwendung eines Proteins N eines Virus der Familie der *Paramyxoviridae*, wobei das lösliche Protein N in Form eines Protein N- geschnittenen Protein P -Komplexes des gleichen Virus der Familie der *Paramyxoviridae* wie in einem der Ansprüche 4 bis 6 definiert ist, wobei das Virus der Familie der *Paramyxoviridae* ein Virus der Unterfamilie der *Pneumovirinae* ist, oder eines Proteins N des RSV gemäß Anspruch 9 zum *in vitro* Nachweis von Antikörpern, die gegen das Protein N gerichtet sind.

15. Nachweisverfahren von Antikörpern, die spezifisch gegen das Protein N eines Virus der Familie der *Paramyxoviridae* gerichtet sind, in einer biologischen Probe, wobei das Verfahren die Schritte bestehend aus:
a) In-Kontakt-bringen der biologischen Probe mit einem Protein N eines Virus der Familie der *Paramyxoviridae*, wobei das Protein N die Form eines Protein N-geschnittenen Protein P Komplexes des gleichen Virus der Familie der *Paramyxoviridae* wie in einem der Ansprüche 4 bis 6 definiert hat,
b) Detektieren der gebildeten Protein N/Antikörper -Komplexe,
umfasst,
wobei die Anwesenheit dieser Komplexe gleichbedeutend mit der Anwesenheit von für das Protein N eines Virus der Familie der *Paramyxoviridae* spezifischen Antikörpern in der biologischen Probe ist,
wobei das Virus der Familie der *Paramyxoviridae* ein Virus der Unterfamilie der *Pneumovirinae* ist.

## Claims

1. Method of preparing a soluble N protein - truncated P protein complex of a virus of the *Paramyxoviridae* family, said method comprising the steps consisting in:
a) coexpressing an N protein of a virus of the *Paramyxoviridae* family with a truncated P protein of the same virus of the *Paramyxoviridae* family, said truncated P protein being devoid of the P oligomerisation domain and being capable of interacting with the N protein;
b) collecting the soluble N protein - truncated P protein complexes thus formed;
wherein the virus of the *Paramyxoviridae* family is a virus of the *Pneumovirinae* subfamily.

2. Method according to claim 1, wherein the *Paramyxoviridae* virus is bovine or human respiratory syncytial virus (RSV).

3. Method according to claim 2, wherein there is coexpressed:
a) a C-terminal fragment of the RSV P protein which comprises the sequence of amino acids 233 to 241 of the P protein of the human RSV LONG strain as shown in SEQ ID No. 1 and which extends in the N-terminal direction up to an amino acid residue between positions 233 and 161 of the sequence of the RSV P protein as shown in SEQ ID No. 1, or
b) a C-terminal fragment, homologous to the fragment defined in a), of a P protein from another strain of human RSV or from a strain of bovine RSV, the C-terminal fragment of the P protein being a chain of amino acids positioned in the C-terminal half of the primary sequence of the P protein, being devoid of the P oligomerisation domain but being capable of interacting with the N protein.

4. Isolated soluble N protein - truncated P protein complex of a virus of the *Paramyxoviridae* family, said N protein being in the form of ribonucleocapside and said virus of the *Paramyxoviridae* family being a virus of the *Pneumovirinae* subfamily, said truncated P protein being devoid of the P oligomerisation domain and being capable of interacting with the N protein.

5. Isolated soluble complex according to claim 4, wherein truncated P protein is
a) a C-teiminal fragment which comprises the sequence of amino acids 233 to 241 of the P protein of the human RSV LONG strain as shown in SEQ ID No. 1 and which extends in the N-terminal direction up to an amino acid residue between positions 233 and 161 of the sequence of the RSV P protein as shown in SEQ ID No. 1, or
b) a C-terminal fragment, homologous to the fragment defined in a), of a P protein from another strain of human RSV or from a strain of bovine RSV, the C-terminal fragment of the P protein being a chain of amino acids positioned in the C-terminal half of the primary sequence of the P protein, being devoid of the P oligomerisation domain but being capable of interacting with the N protein.

6. Isolated soluble complex N protein - C-terminal fragment of the P protein of the respiratory syncytial virus (RSV), comprising 10 molecules of N protein, and wherein said C-terminal fragment of P comprises the last 9 C-terminal amino acids of the RSV P protein and is devoid of at least the 160 N-terminal amino acids of the RSV P protein.

7. Method of preparing soluble N proteins of a virus of the *Paramyxoviridae* family, said method comprising the steps consisting in:
a) preparing a soluble N protein - truncated P protein complex by a method according to any one of claims 1 to 3; and
b) separating the soluble N proteins from the soluble N protein - truncated P protein complexes;
wherein the virus of the *Paramyxoviridae* family is a virus of the *Pneumovirinae* subfamily.

8. Method for the preparation of soluble N proteins according to claim 7, wherein said truncated P protein is a C-terminal fragment of the P protein, the C-terminal fragment of the P protein being a chain of amino acids positioned in the C-terminal half of the primary sequence of the P protein, being devoid of the P oligomerisation domain but being capable of interacting with the N protein.

9. Soluble ribonucleocapsides comprising VRS N proteins associated in rings having a diameter of about 7 nm and containing 10 subunits and further comprising an RNA of about 70 bases.

10. Vaccine composition comprising soluble N proteins of a virus of the *Paramyxoviridae* family, in a pharmaceutically acceptable carrier, said N proteins being in the form of ribonucleocapsides, and said virus of the *Paramyxoviridae* family being a virus of the *Pneumovirinae* subfamily.

11. Vaccine composition according to claim 10, wherein the soluble N proteins of a virus of the *Paramyxoviridae* family are obtainable by a process according to claim 7 or 8.

12. Vaccine composition comprising a soluble N protein - truncated P protein complex according to any one of claims 4 to 6 in a pharmaceutically acceptable carrier.

13. Diagnostic reagent comprising an N protein of a virus of the *Paramyxoviridae* family, wherein said N protein is in the form of a complex N protein - truncated P protein of the same virus of the *Paramyxoviridae* family as defined in any one of claims 4 to 6; said virus of the *Paramyxoviridae* family being a virus of the *Pneumovirinae* subfamily, or a VRS N protein according to claim 9.

14. Use of an N protein of a virus of the *Paramyxoviridae* family, said N protein being in the form of a complex N protein - truncated P protein of the same virus of the *Paramyxoviridae* family as defined in any one of claims 4 to 6, said virus of the *Paramyxoviridae* family being a virus of the *Pneumovirinae* subfamily, or a VRS N protein according to claim 9, for the in vitro detection of antibodies directed against said N protein.

15. Method for the detection, in a biological sample, of antibodies specifically directed against the N protein of a virus of the *Paramyxoviridae* family, said method comprising the steps consisting in:
a) contacting said biological sample with an N protein of a virus of the *Paramyxoviridae* family, said N protein being in the form of a complex N protein - truncated P protein of the same virus of the *Paramyxoviridae* family as defined in any one of claims 4 to 6,
b) detecting the N protein/antibody complexes formed,
the presence of such complexes being indicative of the presence of antibodies specific of the N protein of the virus of the *Paramyxoviridae* family in the biological sample,
wherein said virus of the *Paramyxoviridae* family is a virus of the *Pneumovirinae* subfamily.
